(19) Europäisches Patentamt

European Patent Office

Office européen des brevets

(11) **EP 1 485 432 B1**

(12) **EUROPÄISCHE PATENTSCHRIFT**

(45) Veröffentlichungstag und Bekanntmachung des Hinweises auf die Patenterteilung:
**14.06.2006 Patentblatt 2006/24**

(21) Anmeldenummer: **03708172.6**

(22) Anmeldetag: **04.03.2003**

(51) Int Cl.:
*C08L 33/00* (2006.01)    *C08L 23/00* (2006.01)
*C08L 51/00* (2006.01)    *C08F 265/00* (2006.01)
*A61L 15/24* (2006.01)

(86) Internationale Anmeldenummer:
**PCT/EP2003/002183**

(87) Internationale Veröffentlichungsnummer:
**WO 2003/076514 (18.09.2003 Gazette 2003/38)**

(54) **POLYMERGEMISCHE MIT VERBESSERTER GERUCHSKONTROLLE**

POLYMER MIXTURES WITH IMPROVED ODOR CONTROL

MELANGES POLYMERIQUES AYANT UN MEILLEUR CONTROLE D'ODEUR

(84) Benannte Vertragsstaaten:
**AT BE BG CH CY CZ DE DK EE ES FI FR GB GR HU IE IT LI LU MC NL PT RO SE SI SK TR**

(30) Priorität: **08.03.2002 DE 10210124**

(43) Veröffentlichungstag der Anmeldung:
**15.12.2004 Patentblatt 2004/51**

(73) Patentinhaber: **BASF Aktiengesellschaft**
**67056 Ludwigshafen (DE)**

(72) Erfinder:
• **ENGELHARDT, Friedrich**
**60386 Frankfurt (DE)**
• **FUNK, Rüdiger**
**65527 Niedernhausen (DE)**
• **WANIOR, Mariola**
**63526 Erlensee (DE)**

(56) Entgegenhaltungen:
EP-A- 0 454 497          US-A- 5 645 542
US-A- 5 672 419          US-A- 5 716 707

• DATABASE WPI Section Ch, Week 199602 Derwent Publications Ltd., London, GB; Class A14, AN 1996-017235 XP002251219 & JP 07 292023 A (KURARAY CO LTD), 7. November 1995 (1995-11-07)
• DATABASE WPI Section Ch, Week 199025 Derwent Publications Ltd., London, GB; Class A97, AN 1990-185926 XP002251220 & JP 02 113826 A (KYORITSU YUKI CO LTD), 26. April 1990 (1990-04-26)

**Beschreibung**

Die vorliegende Erfindung betrifft Polymerengemische enthaltend wässrige Flüssigkeiten absorbierende Hydrogel-formende Polymere, hergestellt durch Polymerisation von olefinisch ungesättigten Carbonsäuren oder deren Derivaten, zusammen mit Copolymeren aus $C_2$ - $C_8$ Olefinen oder Styrolen mit Anhydriden, sowie deren Herstellung, Verwendung in Hygieneartikeln und Hygieneartikel, die diese enthalten. Insbesondere betrifft die Erfindung 2-Komponenten-Polymerenmischungen aus Hydrogel-formenden Polymeren mit Copolymeren aus $C_2$ - $C_8$ Olefinen oder Styrolen mit Anhydriden.

Quellbare Hydrogel-bildende Polymerisate, sogenannte Superabsorber (Super-Absorbing Polymers), sind aus dem Stand der Technik bekannt. Hierbei handelt es sich um Netzwerke flexibler hydrophiler Polymerisate, die sowohl ionischer als auch nichtionischer Natur sein können. Diese sind in der Lage, wässrige Flüssigkeiten unter Bildung eines Hydrogels zu absorbieren und zu binden und werden daher bevorzugt für die Herstellung von Tampons, Windeln, Damenbinden, Inkontinenzartikeln, Trainingsunterwäsche für Kinder, Schuheinlagen und anderen Hygieneartikeln bei der Absorption von Körperflüssigkeiten verwendet. Superabsorber werden außerdem in anderen Gebieten der Technik eingesetzt, bei denen Flüssigkeiten, insbesondere Wasser oder wässrige Lösungen absorbiert werden. Diese Gebiete sind z.B. Lagerung, Verpackung, Transport (Verpackungsmaterial wassersensitiver Artikel, wie z.B. Blumentransport, Schock Protection); Lebensmittelsektor (Transport von Fisch, Frischfleisch; Absorption von Wasser, Blut in Frischfisch-/Fleisch-Verpackungen); Medizin (Wundpflaster, wasserabsorbierendes Material für Brandverbände oder für andere nässende Wunden), Kosmetik (Trägermaterial für Pharmachemikalien und Medikamente, Rheumapflaster, Ultraschallgel, Kühlgel, Kosmetikverdicker, Sonnenschutz); Verdicker für Öl/Wasser bzw. Wasser/Öl-Emulsionen; Textil (Handschuhe, Sportbekleidung, Feuchtigkeitsregulation in Textilien, Schuheinlagen); chem.-techn. Anwendungen (Katalysator für org. Reaktionen, Immobilisierung großer funktioneller Moleküle (Enzyme), Adhesiv für Agglomerationen, Wärmespeicher, Filtrationshilfsmittel, hydrophile Komponente in Polymerlaminaten, Dispergiermittel, Verflüssiger); Bau- und Konstruktionswesen, Installation (Pulverspritzguss, lehmbasierende Putze, Vibrationshemmendes Medium, Hilfsmittel bei Tunnelgrabungen in wasserreichem Untergrund, Kabelummantelung); Wasserbehandlung, Abfallbehandlung, Wasserabtrennung (Enteisungsmittel, wiederverwendbare Sandsäcke); Reinigung; Agrarindustrie (Bewässerung, Rückhaltung von Schmelzwasser und Tauniederschläge, Kompostierungszusatz, Schutz der Wälder vor Pilz-/Insektenbefall, verzögerte Freisetzung von Wirkstoffen an Pflanzen); im Feuerschutz (Funkenflug) (Abdecken von Häusern bzw. Bedecken von Hauswänden mit SAP Gel, da das Wasser eine sehr hohe Wärmekapazität hat, kann ein Entflammen verhindert werden; Versprühen von SAP Gel bei Bränden wie z.B. Waldbränden); Coextrusionsmittel in thermoplastischen Polymeren (Hydrophilierung von Mehrschichtfolien); Herstellung von Folien und thermoplastischen Formkörpern, die Wasser absorbieren können (z.B. Regen- und Tauwasser speichernde Folien für die Landwirtschaft; SAP-haltige Folien zum Frischhalten von Obst und Gemüse, die in feuchte Folien verpackt werden können; der SAP speichert vom Obst und Gemüse abgegebenes Wasser ohne Bildung von Kondensationstropfen und gibt das Wasser teilweise an das Obst und Gemüse wieder ab, so dass weder Faulen noch Verwelken einritt; SAP-Polystyrol Coextrudate z.B. für Lebensmittelverpackungen wie Fleisch, Fisch, Geflügel, Obst und Gemüse); Trägersubstanz in Wirkstoffformulierungen (Pharma, Pflanzenschutz). In den Hygieneartikeln befinden sich die Superabsorber in aller Regel im sog. absorbent core, der als weitere Materialien unter anderem Fasern (Cellulosefasern) umfasst, die als eine Art Flüssigkeitsreservoir die spontan beaufschlagten Flüssigkeitsmengen zwischenspeichern und eine gute Kanalisation der Körperflüssigkeiten im absorbent core hin zum Superabsorber gewährleisten sollen.

Der aktuelle Trend im Hygienebereich, z. B. beim Windelaufbau besteht darin, dünnere Konstruktionen mit reduziertem Cellulosefaseranteil und erhöhtem Hydrogelanteil herzustellen. Mit dem Trend zu immer dünner werdenden Windelkonstruktionen hat sich das Anforderungsprofil an die wasserquellbaren hydrophilen Polymeren über die Jahre deutlich verändert. Während zu Beginn der Entwicklung hochsaugfähiger Hydrogele zunächst allein das sehr hohe Quellvermögen in Vordergrund stand, hat sich später gezeigt, dass auch die Fähigkeit des Superabsorbers zur Flüssigkeitsweiterleitung und -verteilung von entscheidender Bedeutung ist. Es hat sich gezeigt, dass herkömmliche Superabsorber an der Oberfläche bei Benetzung mit Flüssigkeit stark anquellen, so dass der Flüssigkeitstransport ins Partikelinnere stark erschwert oder ganz unterbunden wird. Diese Eigenart des Superabsorbers wird auch als "Gelblocking" bezeichnet. Aufgrund der höheren Beladung des Hygieneartikels (Polymer pro Flächeneinheit) darf das Polymer im gequollenen Zustand keine Sperrschicht für nachfolgende Flüssigkeit bilden. Weist das Produkt gute Transporteigenschaften auf, so kann eine optimale Ausnutzung des gesamten Hygieneartikels gewährleistet werden. Das Phänomen des Gelblockings wird somit unterbunden, das im Extremfall zum Austritt der Flüssigkeit, der sog. Leakage des Hygieneartikels führt. Die Flüssigkeitsweiterleitung und -verteilung ist also bei der anfänglichen Absorption von Körperflüssigkeiten von entscheidender Bedeutung.

In einer Vielzahl von Arbeiten wurde versucht, absorbierende Strukturen zu generieren, die keinerlei Zusätze von Cellulosefasern oder anderen Nonwoven Fasermaterialien aufweisen, die im Idealfall sogar kontinuierliche Hydrogelzonen ausbilden um eine höhere Beladung des absorbent cores mit hochsaugfähigem Hydrogelbildendem Polymermaterial zu gewährleisten.

In der Literatur findet man darüber hinaus den Einsatz wasserabsorbierender Filme, denen ebenfalls Hydrogel-bildende Polymerisate zugrunde liegen, deren Monomerlösung allerdings vor der Polymerisation flächig aufgetragen wird, oder aber, ausgehend von Hydrogel-bildenden Polymerpartikeln, eine intramolekulare Vernetzung zu Makrostrukturen durchgeführt wird. So beschreibt JP 04004247 die Herstellung eines wasserabsorbierenden Filmes aus Maleinsäure-Anhydrid-Copolymer, dessen strukturelle Einheiten auf (I) alpha-olefinischen oder Styrol-Einheiten und (II) auf Maleinsäure-Anhydrid Strukturen basieren.

Allerdings kommt es bei Einsatz wasserabsorbierender Filme zu Problemen beim Flüssigkeitstransport, da aufgrund unzureichender Diffusionszeiten durch das Hydrogel eine Flüssigkeitsweiterleitung in untere Schichten erschwert oder sogar unterbunden wird. Dasselbe gilt bei Einsatz Hydrogel-bildender Polymerpartikel in hoher Konzentration, bei dem es beim Anquellvorgang zur gegenseitigen Berührung der gequollenen Hydrogelpartikel kommt, und so eine gelkontinuierliche Zone gebildet wird.

Gute Transporteigenschaften besitzen beispielsweise Hydrogele, die im gequollenen Zustand eine hohe Gelfestigkeit aufweisen. Gele mit nur geringer Gelfestigkeit sind unter einem angewendetem Druck (Körperdruck) deformierbar, verstopfen Poren in dem Superabsorber / Cellulosefaser-Saugkörper und verhindern dadurch eine weitere Flüssigkeitsaufnahme. Eine erhöhte Gelfestigkeit wird in aller Regel durch eine höhere Vernetzung erreicht, wodurch allerdings die Retention des Produktes verringert wird. Entsprechend der Natur Hydrogel-bildender Polymerisate, gelang es nicht, Eigenschaften wie hohe Absorptionskapazität und hohe Gelstärke in einem Produkt zu vereinen.

Eine Möglichkeit zur Erhöhung der Gelfestigkeit unter Wahrung hoher Absorptionskapazitäten stellt die Oberflächennachvernetzung dar. Bei diesem Verfahren werden getrocknete Superabsorber mit durchschnittlicher Vernetzungsdichte einer zusätzlichen Vernetzung unterworfen. Der Prozess ist dem Fachmann bekannt und z. B. in Patent EP-A-0 349 240 beschrieben. Durch die Oberflächennachvernetzung steigt die Vernetzungsdichte in der Schale der Superabsorberpartikel, wodurch die Absorption unter Druckbelastung auf ein höheres Niveau gehoben wird. Während die Absorptionskapazität in der Superabsorberschale sinkt, weist der Kern der Superabsorberpartikel durch das Vorhandensein beweglicher Polymerketten eine im Vergleich zur Schale verbesserte Absorptionskapazität auf, so dass durch den Schalenaufbau eine verbesserte Flüssigkeitsweiterleitung gewährleistet wird, ohne dass der Effekt des Gelblockings auftritt. Es ist durchaus wünschenswert, dass die Gesamtkapazität des Superabsorbers nicht spontan, sondern zeitversetzt ausgeschöpft wird. Da der Hygieneartikel in der Regel mehrmals mit Urin beaufschlagt wird, muss das Absorptionsvermögen des Superabsorbers sinnvoller weise nicht nach der ersten Disposition erschöpft sein. Allerdings verbleibt weiterhin das Problem unzureichender Akquisitionszeiten, die vor allem in den Bereichen des Hygieneartikels optimiert werden muss, in denen die meiste Flüssigkeit beaufschlagt wird.

Finden Hydrogele im Hygienebereich Anwendung, so werden sie Körperflüssigkeiten wie Urin oder Menstruationsblut ausgesetzt. Die Körperflüssigkeiten enthalten generell unangenehm riechende Komponenten vom Amin- oder Fettsäure-Typ, die neben den ohnehin vorhandenen organischen Komponenten (wie beispielsweise Amine, Säuren, Alkohole, Aldeyde, Ketone, Phenole, Polycyclen, Indole Aromaten, Polyaromaten usw.), die für unangenehme Körpergerüche verantwortlich sind, in Erscheinung treten. Die Geruchsentwicklung findet in zwei Stufen statt: als erstes beim Austritt aus der Körperregion, und dann, wenn die Flüssigkeit bereits eine definierte Zeit lang im Absorptionsmedium vorliegt. Beide Geruchsfaktoren gilt es zu eliminieren, da es aus Kostengründen unerwünscht ist, den Hygieneartikel nach jedem Absorptionsvorgang zu wechseln.

Für die Verwendung der hochquellfähigen Hydrogele auf dem Hygienesektor werden derzeit Polymerisate eingesetzt, die einen Neutralisationsgrad zwischen 60 und 80 Mol%, bezogen auf die polymerisierten, Säuregruppen enthaltenden Monomereinheiten, besitzen. Bei der Geruchskontrolle wurde allerdings gefunden, dass generell ein höherer pH-Wert das Bakterienwachstum begünstigt. Dabei wird der Harnstoff im Urin verstärkt durch Urease in Kohlendioxid und Ammoniak gespalten, was zu einer weiteren Erhöhung des pH-Wertes führt. Dies wiederum verstärkt das Bakterienwachstum, und die Enzymtätigkeit wird weiter erhöht. Als Folge des erhöhten pH-Wertes kommt es zu Hautaufweichungen, die dadurch anfälliger für Bakterienbefall wird. Unmittelbare Folge davon sind Hautirritationen, die ein längere Tragezeit des Hygieneartikels ausschließen.

Die Geruchsbindung beim Einsatz von sauren Hydrogelen in Hygieneartikeln ist gut. Allerdings zeigen sich bereits im Herstellungsverfahren nach dem Stand der Technik Nachteile auf, da die Polymerisation der Monomerlösung sehr langsam verläuft, so dass nur eine diskontinuierliche Verfahrensweise in Frage kommt. Außerdem treten erhebliche Probleme bei der Zerteilung des völlig sauren Polymerisatgeles auf, und die Neutralisation im Anschluss erfolgt lediglich diffusionskontrolliert, so dass die Polymerisatoberfläche einen Basenüberschuss aufweist.

Bisher stehen bei der Geruchskontrolle auf dem Hygienesektor folgende Möglichkeiten zur Auswahl:

- Geruchskontrolle bei gleichzeitiger Absorption durch Zugabe inerter anorganischer Substanzen mit großer Oberfläche, in aller Regel als Feststoff auf die Oberfläche von Pulvern oder Granulaten zur Konfektionierung absorbierender Polymerer. Hier gelangen Zeolithe, Aktivkohle, Bentonite, feinteilige amorphe Polykieselsäuren (Silica) wie AEROSIL® oder CAB-O-SIL® zum Einsatz.

- Zugabe von Substanzen, die unter Komplexbildung mit organischen Molekülen oder mit Metallionen, die in der Körperflüssigkeit vorliegen, reagieren, und die dadurch die Entwicklung unangenehmer Gerüche verhindern. Dies geschieht bevorzugt durch den Einsatz von Cyclodextrinen (jede Modifikation unsubstituierter Cyclodextrine, die 6 bis 12 Glucoseeinheiten enthält, wie beispielsweise alpha-Cyclodextrin, beta-Cyclodextrin, gamma-Cyclodextrin und / oder ihren Derivaten und / oder Mischungen daraus). Mischungen von Cyclodextrinen sind bevorzugt, da eine breitere Komplexierung organischer Moleküle über einen weiteren Molekulargewichtsbereich erfolgt. Cyclodextrine werden von 0.1% bis etwa 25 %, bevorzugt von 1% bis etwa 20 %, besonders bevorzugt von 2 % bis etwa 15 %, insbesondere bevorzugt von 3 bis 10 %, bezogen auf das Gesamtgewicht der Zusammensetzung, eingesetzt. Cyclodextrine werden in kleiner Partikelgröße (meist kleiner als 12 $\mu$m) zugesetzt, um zur Geruchseliminierung eine große Oberfläche anzubieten. Weitere Komplexbildner: Aminopolycarbonsäuren und ihre Salze, Ethylendiamintetraacetat EDTA Ethylendiaminpentamethylenphosphonsäure, Ethylendiamintetramethylenphosphonsäure, Aminophosphate, polyfunktionelle Aromaten, N,N-Disuccinsäure.

- Maskierung unangenehmer Gerüche durch Zusatz von Parfüms bzw. Deodorants. Diese werden in freier Form oder in eingekapselter Form (beispielsweise in Cyclodextrinen) zugesetzt. Letztere Form erlaubt es, das Parfüm zeitverzögert freizusetzen. Beispiele von Parfüms sind, ohne sich jedoch darauf zu beschränken: Allylcaproat, Allylcyclohexanacetat, Allylcyclohexanpropionat, Allylheptanoat, Amylacetat, Amylpropionat, Anetol, Anisol, Benzaldehyd, Benzylacetat, Benzylaceton, Benzylalkohol, Benzylbutyrat, Benzylformat, Benzylisovalerat, Benzylpropionat, Butylbenzoat, Butylcaproat, Campher, cis-3-Hexenylacetat, cis-3-Hexenylbutyrat, cis-3-Hexenylcaproat, cis-3-Hexenylvalerat, Zitronellol, Zitronellylderivate, Cyclal C, Cyclohexylethylacetat, 2-Decenal, Decylaldehyd, Dihydromyrcenol, Dimethylbenzylcarbinol und deren Derivate, Dimethyloktanol, Diphenyloxid, Ethylacetat, Ethylacetoacetat, Ethylamylketon, Ethylbenzoat, Ethylbutyrat, Ethylhexylketon, Ethylphenylacetat, Eucalyptol, Fenchylacetat, Fenchylalkohol, Tricyclodecenylacetat, Tricyclodecenylpropionat, Geraniol, Geranyl-Derivate, Heptylacetat, Heptylisobutyrat, Heptylpropionat, Hexenol, Hexenylacetat, Hexenylisobutyrat, Hexylacetat, Hexylformat, Hexylisobutyrat, Hexylisovalerat, Hexylneopentanoat, Hydroxycitronellal, $\alpha$-Ionon, $\beta$-Ionon, $\gamma$-Ionon, Isoamylalkohol, Isobornylacetat, Isobornylpropionat, Isobutylbenzoat, Isobutylcaproat, Isononylacetat, Isononylalkohol, Isomenthol, Isomenthon, Isononylacetat, Isopulegol, Isopulegylacetat, Isoquinolin, Dodecanal, Lavandulylacetat, Ligustral, $\delta$-Limonen, Linalool und Derivate, Menthon, Menthylacetat, Methylacetophenon, Methylamylketon, Methylanthranilat, Methylbenzoat, Methylbenzylacetat, Methylchavicol, Methyleugenol, Methylheptenon, Methylheptincarbonat, Methylheptylketon, Methylhexylketon, Methylnonylacetaldehyd, $\alpha$-iso"$\gamma$"Methylionon, Methyloktylacetaldehyd, Methyloktylketon, Methylphenylcarbinylacetat, Methylsalicylat, Myrcen, Myrcenylacetat, Neral, Nerol, Nerylacetat, Nonalakton, Nonylbutyrat, Nonylalkohol, Nonylacetat, Nonylaldehyd, Oktalakton, Oktylacetat, Oktylalkohol, Oktylaldehyd, D-Limonen, p-Kresol, p-Kresylmethylether, p-Kymene, p-Isopropylp-Methylacetophenon, Phenethylanthranilat, Phenoxyethanol, Phenylacetaldehyd, Phenylethylacetat, Phenylethylalkohol, Phenylethyldimethylcarbinol, $\alpha$-Pinen, $\beta$-Pinen, $\alpha$-Terpinen, $\gamma$-Terpinen, Terpineol, Terpinylacetat, Terpinylpropionat, Tetrahydrolinalool, Tetrahydromyrcenol, Thymol, Prenylacetat, Propylbutyrat, Pulegon, Safrol, $\delta$-Undecalakton, $\gamma$-Undecalakton, Undecanal, Undecylalkohol, Veratrol, Verdox, Vertenex, Viridin.

- Zugabe von Ureaseinhibitoren, die die Bildung oder Aktivität von Enzymen unterbinden, die für die Spaltung von Harnstoff in Ammoniak und somit für die Geruchsentwicklung verantwortlich zeichnen.

- Zugabe antimikrobieller Substanzen. Enzyme kontrollieren das bakterielle Wachstum und minimieren dadurch die Geruchsentwicklung, die über bakterielle Abbauvorgänge entsteht (z.B. Oxidoreductase + Mediator). Beispiele antimikrobieller Substanzen schließen quaternäre Ammoniumverbindungen, Phenole, Amide, Säuren und Nitroverbindungen, sowie deren Mischungen mit ein.

Beispiele für quaternäre Ammoniumverbindungen schließen 2-(3-anilinovinylul)3,4-dimethyloxazoliniumiodid, Alkylisoquinoliumbromid, Benzalkoniumchlorid, Benzethoniumchlorid, Cetylpyridiniumchlorid, Chlorhexidingluconat, Chlorhexidinehydrochlorid, Lauryltrimethylammonium-Verbindungen, Methylbenzethoniumchlorid, Stearyltrimethylammoniumchlorid, 2,4,5-Trichlorophenoxid, sowie deren Mischungen mit ein.

Beispiele für Phenole schließen Benzylalkohol, p-Chlorophenol, Chlorocresol, Chloroxylenol, Cresol, o-Kymene-5-ol (BIOSOL), Hexachlorophen, Chinokitiol, Isopropylmethylphenol, Parabene (mit Methyl, Ethyl, Propyl, Butyl, Isobutyl, Isopropyl, und/oder Natrium-methyl-Substituenten), Phenethylalkohol, Phenol, Phenoxyethanol, o-Phenylphenol, Resorcin, Resorcinmonoacetat, Natriumparabene, Natriumphenolsulfonat, Thioxolon, 2,4,4'-trichloro-2'-hydroxidiphenylether, Zinkphenolsulfonat, Di-tert.-butylphenol, Hydrochinon; BHT, sowie deren Mischungen mit ein.

Beispiele für Amide schließen Diazolidinylurea, 2,4-imidazolidindi-on (HYDATOIN), 3,4,4'-Trichlorocarbanilid, 3-Trifluoromethyl-4,4'-dichlorocarbanilid, Undecylensäuremonoethanolamid, sowie deren Mischungen mit ein.

Beispiele für Säuren schließen Benzoate, Benzoesäure, Zitronensäure, Dehydroessigsäure, Kaliumsorbat, Natriumcitrate, Natriumdehydroacetat, Natriumsalicylat, Natriumsalicylsäure, Sorbitansäure, Undecylensäure, Zinkundecylenat, Zinkoxid, Zinkphenolsulfonat, Ascorbinsäure, Acetylsalicylsäure, Salicylaldehyd, Salicylsäurederivate, Adipinsäure, Adipinsäurederivate, sowie deren Mischungen mit ein.

Beispiele für Nitroverbindungen schließen

2-Bromo-2-Nitro-2,3-Propandiol (BRONOPOL), Methyldibromoglutaronitril und Propyuleneglycol (MERGUARD), sowie deren Mischungen mit ein.

Daneben kommen noch folgende Verbindungen als Biozide in Frage: 2,5-Dimethoxytetrahydrofuran, 2,5-Diethoxytetrahydrofuran, 2,5-Dimethoxy-2,5-dihydrofuran, 2,5-Diethoxy-2,5-dihydrofuran, Succindialdehyd, Glutardialdehyd, Glyoxal, Glyoxylsäure, Hexahydrotriazin, Tetrahydro-3,5-dimethyl-2H-1,3,5-thiadiazine-2-thione (Dazomet), 2,4-Dichlorbenzylalkohol, Benzalkoniumchlorid, Chlorhexidingluconat, Triclosan.

- Einsatz von Übergangsmetallverbindungen (Cu, Ag, Zn).
- Einsatz von Mikrokapseln, welche die Aktivsubstanz bei Feuchtigkeit freisetzen;

Neben den bezeichneten Verbindungsklassen gelangen bei der Geruchskontrolle noch folgende Verbindungen zum Einsatz: Peroxide, Hydrogencarbonat, Triclosan, Pflanzenextrakte, etherische Öle, Borverbindungen, Poly-Alpha-Aminosäuren, (Polylysin), Imide, Polyimide, PVP-Jod, Verwendung bestimmter polymerer Substanzen wie Chitosan, Polyglycoside, Oxidationsmittel, Cyclophane.

Allgemein wirkt sich allerdings der Zusatz geruchsinhibierender Stoffe nachteilig auf das Absorptionsprofil der hochquellfähigen Hydrogele aus. Daher werden die erfindungsgemäßen Polymerenmischungen vorzugsweise ohne diese geruchsinhibierenden Stoffe verwendet.

Es bestand nun die Aufgabe, ein Produkt mit der Zielsetzung zu entwickeln, bei hoher Absorptionsleistung und/oder Quellgeschwindigkeit gleichzeitig geruchsbindende Eigenschaften aufzuweist.

Es wurde überraschend gefunden, dass eine gute Geruchsbindung erreicht werden kann, wenn man üblichen Hydrogel-bildenden Polymerisaten Copolymere aus $C_2$ - $C_8$ Olefinen oder Styrolen mit Anhydriden zusetzt.

Die vorliegende Erfindung betrifft demnach Polymergemische enthaltend wässrige Flüssigkeiten absorbierende Hydrogel-formende Polymere, hergestellt durch Polymerisation von olefinisch ungesättigten Carbonsäuren oder deren Derivaten (Komponente (i)), mit Copolymeren aus $C_2$ - $C_8$ Olefinen oder Styrolen mit Anhydriden (Komponenten (ii)). Die erfindungsgemäßen Polymergemische betreffen vorzugsweise granulären oder faserförmigen wässrige Flüssigkeiten absorbierende Hydrogel-formende Polymere, hergestellt durch Polymerisation von olefinisch ungesättigten Carbonsäuren oder deren Derivate, in Kombination mit granulärem oder faserförmigem Copolymeren aus $C_2$ - $C_8$ Olefinen oder Styrolen mit Anhydriden. Alternativ können die Copolymeren aus $C_2$ - $C_8$ Olefinen oder Styrolen mit Anhydriden auch auf die granulären oder faserförmigen wässrige Flüssigkeiten absorbierenden Hydrogel-formende Polymere, hergestellt durch Polymerisation von olefinisch ungesättigten Carbonsäuren oder deren Derivaten, aufgesprüht werden. Unter granulären oder faserförmigen wässrige Flüssigkeiten absorbierenden Hydrogel-formende Polymere, hergestellt durch Polymerisation von olefinisch ungesättigten Carbonsäuren oder deren Derivaten werden bei der vorliegenden Erfindung keine Copolymeren aus $C_2$ - $C_8$ Olefinen oder Styrolen mit Anhydriden verstanden. Bevorzugt werden die wässrige Flüssigkeiten absorbierenden Hydrogel-formende Polymere, hergestellt durch Polymerisation von Acrylsäure oder deren Salze. Diese wässrige Flüssigkeiten absorbierenden Hydrogel-formende Polymere sind daher bevorzugt auf Polyacrylatbasis. Die Copolymere aus $C_2$ - $C_8$ Olefinen oder Styrolen mit Anhydriden können selbst Hydrogel-formend sein, z.B. in dem sie teilverseift vorliegen. Dabei liegen bevorzugt 15 mol-% oder weniger teilverseift vor. Besonders bevorzugt sind unverseifte Coploymere. Bevorzugt werden Polymergemische hergestellt durch ein Verfahren bei dem das Hydrogelbildende Polymerisate und das Copolymere aus $C_2$ - $C_8$ Olefinen oder Styrolen mit Anhydriden in zwei Verfahrensschritten hergestellt und anschließend in definiertem Verhältnis gemischt werden.

Die Copolymeren aus $C_2$ - $C_8$ Olefinen oder Styrolen mit Anhydriden werden bevorzugt unverseift in Pulverform (granulär) eingesetzt. Wird dieses Copolymer im Hygienebereich eingesetzt, so kommt es unter Ringöffnung der Anhydrid-Komponente zur Aufnahme des durch enzymatische Prozesse oder bakterielle Abbaureaktionen gebildeten basischen Komponenten, z.B. Ammoniaks, die im wesentlichen für die Geruchsentwicklung verantwortlich sind. Unter der Anhydrid Komponente werden erfindungsgemäß bevorzugt Anhydride von olefinisch ungesättigte Di- oder Polycarbonsäuren verstanden, z.B. durch eine oder zwei $C_1$-$C_6$-Alkylgruppen substituierte Maleinsäure. Di-Carbonsäuren sind bevorzugt. Maleinsäureanhydrid ist besonders bevorzugt. Unter den $C_2$ - $C_8$ Olefinen werden ungesättigte Verbindungen verstanden, die 2 bis 8 C-Atome enthalten. Optional könne auch 1 oder mehrere Heteroatome wie O, N, S enthalten sein. Beispielsweise werden folgende Monomere verstanden Ethylen, Propylen, Isobutylen, 1-Butylen, $C_1$-$C_4$-Methacrylate., Vinylacetat, Methylvinylether, Isobutylvinylether, 1-Hexen. Die Monomere können rein oder in Mischungen vorliegen. Bevorzugt sind Isobutylen und Vinylacetat. Unter Styrolen werden Styrol und substituierte Styrole verstanden. Am alpha Kohlenstoff kann z.B. eine $C_1$-$C_6$-Alkylgruppe vorliegen und/oder der Benzolring kann mit einer oder mehreren

$C_1$-$C_6$-Alkylgruppen und/oder ein oder mehreren Hydroxygruppen substituiert sein. Bevorzugt ist Styrol. Das molare Verhältnis zwischen Anhydrid und Olefin bzw. Styrol liegt zwischen 3 zu 1 und 1 zu 3, bevorzugt zwischen 2,5 zu 1 und 1 zu 2,5, besonders bevorzugt zwischen 2 zu 1 und 1 zu 2, insbesondere bei 1 zu 1. Wenn ein Überschuss einer Komponente vorliegt ist ein Anhydrid Überschuss bevorzugt. Die Copolymere mit Maleinsäureanhydrid und Isobutylen, Diisobutylen, Ethylen oder Styrol, alle optional mit Zusatz von Vinylacetat, sind besonders bevorzugt.

Überraschenderweise hat sich zudem gezeigt, dass durch Zusatz von granulären oder faserförmigen Copolymeren aus $C_2$ - $C_8$ Olefinen oder Styrolen mit Anhydriden zu den Hydrogel-bildenden Polymerisaten aus dem Stand der Technik die Permeabilität weitgehend gesteigert werden kann.

Die wässrige Flüssigkeiten absorbierende Hydrogel-formende Polymere können auch mit Monomerlösung oder Polymerlösung von $C_2$ - $C_8$ Olefinen oder Styrolen mit Anhydriden versetzt werden. Als Lösemittel eignen sich inerte Lösemittel wie Aceton, DMSO, Dioxan, Ethylacetat, Chloroform oder Toluol. Die Monomer- oder Polymer-Lösungen können auf die Hydrogele gesprüht werden und gegebenenfalls einer Fällungspolymerisation unterzogen werden. Das Lösemittel wird zweckmäßigerweise durch Trocknen bei 20 bis 120 °C entfernt.

Die Molmassen der Copolymere liegt im Allgemeinen zwischen 500 und 1 Million, vorzugsweise zwischen 1000 und 250.000.

Der Anteil an Hydrogel-bildenden Polymerpartikeln weist eine hohe Absorptionskapazität bei guter Quellgeschwindigkeit auf, während der Erhalt der Anhydrideinheiten die Pufferung des Anteils an basischen Komponenten, z.B. des Ammoniakanteils gewährleistet. Durch die Beigabe Copolymere aus $C_2$ - $C_8$ Olefinen oder Styrolen mit Anhydriden kann zudem eine verbesserte Permeabilität erreicht werden.

In einer bevorzugten Ausführungsform der vorliegenden Erfindung werden den Hydrogel-bildenden Polymerisaten Copolymere aus $C_2$ - $C_8$ Olefinen oder Styrolen mit Anhydriden zugesetzt, die in Faserform vorliegen. Um ein Verspinnen zu ermöglichen, wird das Copolymere aus $C_2$ - $C_8$ Olefinen oder Styrolen mit Anhydriden teilverseift. Derartige teilneutralisierte Copolymere aus $C_2$ - $C_8$ Olefinen oder Styrolen mit Anhydriden sind beispielsweise in US 5,026,784 beschrieben. Danach wird eine wässrige, zum Verspinnen geeignete Copolymerlösung erhalten aus (Spalte 3, Zeile 6) a) teilneutralisiertem $C_2$ - $C_8$ Olefin / Anhydrid-, insbesondere Maleinsäureanhydrid-Copolymer mit einem Neutralisationsgrad von 0.2 bis etwa 0.8 Äquivalenten an Carboxylgruppeneinheiten mit b) 0.1 bis 10 Gewichtsteile mindestens einer reaktiven Komponente bezogen auf 100 Gewichtsteile teilneutralisierten in wässriger Flüssigkeit gelösten Polymers. Bei der reaktiven Komponente handelt es sich um wasserlösliche Komponenten, die eine Aminogruppe und mindestens eine Hydroxylgruppe aufweisen. Das Reaktionsprodukt weist eine ionische Ammoniumcarboxylatbindung auf, die durch unneutralisierte Carboxylgruppen des Polymers und der Aminogruppe der Reaktivsubstanz gebildet wird.

Nach dem Verspinnen werden die Fasern auf 140 bis 210 °C erhitzt, wobei unter Wasseraustritt eine Vernetzung stattfindet, bei der Ester- und Amidbindungen ausgebildet werden. Die so vernetzten Fasern sind wasserquellbar und somit absorbierend.

Der Zusatz teilneutralisierter Copolymere aus $C_2$ - $C_8$ Olefinen oder Styrolen mit Anhydriden in Faserform kann höhere Akquisitionsraten sowie höhere Retentionswerte ermöglichen, als es bei der erfindungsgemäßen granulären Polymermischung der Fall ist. Bei der Teilverseifung werden Anhydridgruppen verbraucht, daher werden im Allgemeinen entweder mehr Copolymeranteile verwendet und/oder bekannte Geruchsinhibitoren zugesetzt. Der Teilverseifungsgrad liegt bevorzugt bei oder unter 15 mol-%.

Eine besonders bevorzugte Ausführungsform der vorliegenden Erfindung betrifft dementsprechend Polymermischungen, enthaltend wässrige Flüssigkeiten absorbierende Hydrogel-formende Polymere, hergestellt durch Polymerisation von olefinisch ungesättigten Carbonsäuren oder deren Derivaten, mit Copolymeren aus $C_2$ - $C_8$ Olefinen oder Styrolen mit Anhydriden (granulär oder durch Besprühen), sowie teilverseiften $C_2$ - $C_8$ Olefin / Anhydrid-, insbesondere Maleinsäureanhydrid-Copolymer - Fasern.

Polymerenmischungen können Mischungen von trocknen wässrige Flüssigkeiten absorbierende Hydrogelformende Polymeren, die hergestellt werden können durch Polymerisation von olefinisch ungesättigten Carbonsäuren oder deren Derivaten, mit trocknen granulären Copolymeren aus $C_2$ - $C_8$ Olefinen oder Styrolen mit Anhydriden sein. Erstere können auch einen Restwassergehalt aufweisen, der geringer ist als ihre jeweilige CRC. Bevorzugt ist der Restfeuchtegehalt geringer als das Eigengewicht des Superabsorbers, mehr bevorzugt geringer als 30 Gew.-% Restfeuchte, insbesondere weniger als 10 Gew.-% Restfeuchte. Unter olefinisch ungesättigten Carbonsäuren werden bevorzugt monoethylenisch ungesättigte Monomere verstanden. Unter deren Derivaten werden Salze, Ester, z.B. $C_1$-$C_6$-Alkylester, Anhydride etc. verstanden, die zu den freien Säuren hydrolysiert werden können.

Bevorzugte Polymerenmischungen sind dadurch gekennzeichnet, dass es sich dabei um Pulvermischungen von wässrige Flüssigkeiten absorbierende Hydrogel-formende Polymeren (Komponente (i)) mit Copolymere aus $C_2$ - $C_8$ Olefinen oder Styrolen mit Anhydriden (Komponente (ii)) handelt.

Ebenfalls bevorzugt sind Polymerenmischungen, dadurch gekennzeichnet, dass die Komponente (i) zu 99,7 Gew.% bis 85 Gew.% und die Komponente (ii) zu 0,3 Gew.% bis 15 Gew.% vorliegt, insbesondere solche bei denen die Komponente (ii) zu 0,5 Gew.% bis 10 Gew.% vorliegt, d.h. z.B. zu 9, 8, 7, 6, 5, 4, 3, 2, 1, oder dazwischen liegenden Gew-%-anteilen. Die weitere oder weiteren Komponenten ergeben dann jeweils 100 Gew.-%.

In einer besonders bevorzugten Ausführung der vorliegenden Erfindung handelt es sich bei Komponente (ii) um Mischungen aus granulärem $C_2$ - $C_8$ Olefin / Anhydrid-, insbesondere Maleinsäureanhydrid-Copolymer und wässrige Flüssigkeiten absorbierenden $C_2$ - $C_8$ Olefin / Anhydrid-, insbesondere Maleinsäureanhydrid-Copolymer-Fasern.

Ebenfalls bevorzugt sind Polymerenmischungen, dadurch gekennzeichnet, dass granuläres $C_2$ - $C_8$ Olefin / Anhydrid-, insbesondere Maleinsäureanhydrid-Copolymer (A) und wässrige Flüssigkeiten absorbierende $C_2$ - $C_8$ Olefin / Anhydrid-, insbesondere Maleinsäureanhydrid-Copolymer-Fasern (B) als die beiden Bestandteile der Komponente (ii), zu 50 Gew.% bis 90 Gew.% Bestandteil (A) und 10 Gew.% bis 50 Gew.% Bestandteil (B) oder dazwischen liegenden Gew-%-anteilen vorliegt. Die weitere oder weiteren Komponenten ergeben dann jeweils zusammen mit den beiden Hauptbestandteilen 100 Gew.-%.

Die genannten Polymerenmischungen können je nach Anwendung dadurch gekennzeichnet sein, dass die Komponenten der Mischung aus Partikeln der gleichen oder unterschiedlicher Kornfraktion hergestellt werden. Bevorzugt sind die der gleichen Kornfraktion.

Die einzelnen Komponenten werden nach der optionalen Oberflächennachvernetzung der Komponente (i) gemischt.

Außerdem werden verschiedene Anwendungen für die Polymermischungen als Absorptionsmittel für wässrige Flüssigkeiten, Dispersionen und Emulsionen offenbart, insbesondere verschiedene Hygieneartikelkonstruktionen enthaltend obige Polymermischungen. Besonders bevorzugt ist die Verwendung der erfindungsgemäßen von Polymerenmischungen als Absorptionsmittel für wässrige Flüssigkeiten bei verminderter Geruchsbildung. Verminderte Geruchsbildung bedeutet, dass durch den Zusatz der Copolymere zu 10 Gew.% die Pufferkapazität um mindestens 0,2 pH-Einheiten, bevorzugt mindestens 0,5 pH-Einheiten, besonders bevorzugt mindestens 0,7 pH-Einheiten, insbesondere mindestens 1,0 pH-Einheiten verbessert wird.

Verfahren zur Herstellung

Hydrogel-formende Polymere

Die Herstellung der wasserquellbaren hydrophilen Hydrogel-bildenden Polymere erfolgt in der Regel durch radikalische Polymerisation in wässriger Lösung, die die Monomeren, sowie gegebenenfalls Pfropfgrundlage und Vernetzer enthält.

Eingesetzte Monomere

Hydrogel-formende Polymere sind insbesondere Polymere aus (co-)polymerisierten hydrophilen Monomeren, Pfropf(co-)polymere von einem oder mehreren hydrophilen Monomeren auf eine geeignete Pfropfgrundlage, vernetzte Cellulose- oder Stärkeether, vernetzte Carboxymethylcellulose, teilweise vernetztes Polyalkylenoxid oder in wässrigen Flüssigkeiten quellbare Naturprodukte, wie beispielsweise Guarderivate, Alginate und Carrageenane.

Geeignete Pfropfgrundlagen können natürlichen oder synthetischen Ursprungs sein. Beispiele sind Stärke, Cellulose oder Cellulosederivate, wie z.B. Carboxymethylcellulose, sowie andere Polysacharide und Oligosacharide, Polyvinylalkohol, Polyalkylenoxide, insbesondere Polyethylenoxide und Polypropylenoxide, Polyamine, Polyamide sowie hydrophile Polyester. Geeignete Polyalkylenoxide haben beispielsweise die Formel $R^1$-O-$(CH_2$-CHX-O-$)_n R^2$ mit

$R^1$ und $R^2$      unabhängig voneinander Wasserstoff, Alkyl, Alkenyl oder Aryl,

X      Wasserstoff oder Methyl und

n      eine ganze Zahl von 1 bis 10000 bedeuten.

$R^1$ und $R^2$      bedeuten bevorzugt Wasserstoff, $(C_1$ - $C_4)$-Alkyl, $(C_2$ - $C_6)$-Alkenyl oder Phenyl.

Bevorzugt sind als Hydrogel-formende Polymere vernetzte Polymere mit Säuregruppen, die überwiegend in Form ihrer Salze, in der Regel Alkali- oder Ammoniumsalze, vorliegen. Derartige Polymere quellen bei Kontakt mit wässrigen Flüssigkeiten besonders stark zu Gelen auf.

Bevorzugt sind Polymere, die durch vernetzende Polymerisation oder Copolymerisation von säuregruppentragenden monoethylenisch ungesättigten Monomeren oder deren Derivate, z.B. Salze, Ester, Anhydride erhalten werden. Ferner ist es möglich, diese Monomere ohne Vernetzer zu (co-)polymerisieren und nachträglich zu vernetzen.

Solche Säuregruppen tragenden Monomere sind beispielsweise monoethylenisch ungesättigte $C_3$- bis $C_{25}$-Carbonsäuren oder Anhydride wie Acrylsäure, Methacrylsäure, Ethacrylsäure, $\alpha$-Chloracrylsäure, Crotonsäure, Maleinsäure, Maleinsäureanhydrid, Itaconsäure, Citraconsäure, Mesaconsäure, Glutaconsäure, Aconitsäure und Fumarsäure.

Weiterhin kommen monoethylenisch ungesättigte Sulfon- oder Phosphonsäuren in Betracht, beispielsweise Vinylsulfonsäure, Allylsulfonsäure, Sulfoethylacrylat, Sulfomethacrylat, Sulfopropylacrylat, Sulfopropylmethacrylat, 2-Hydroxy-3-acryloxypropylsulfonsäure, 2-Hydroxy-3-methacryl-oxypropylsulfonsäure, Vinylphosphonsäure, Allylphosphonsäure, Styrolsulfonsäure und 2-Acryl-amido-2-methylpropansulfonsäure. Die Monomeren können allein oder in Mischung untereinander eingesetzt werden.

Bevorzugt eingesetzte Monomere sind Acrylsäure, Methacrylsäure, Vinylsulfonsäure, Acrylamidopropansulfonsäure oder Mischungen dieser Säuren, z. B. Mischungen aus Acrylsäure und Methacrylsäure, Mischungen aus Acrylsäure und Acrylamidopropan-sulfonsäure oder Mischungen aus Acrylsäure und Vinylsulfonsäure.

Zur Optimierung von Eigenschaften kann es sinnvoll sein, zusätzliche monoethylenisch ungesättigte Verbindungen einzusetzen, die keine Säuregruppen tragen, aber mit den säuregruppentragenden Monomeren copolymerisierbar sind. Hierzu gehören beispielsweise die Amide und Nitrile von monoethylenisch ungesättigten Carbonsäuren, z. B. Acrylamid, Methacrylamid und N-Vinylformamid, N-Vinylacetamid, N-Methyl-vinylacetamid, Acrylnitril und Methacrylnitril. Weitere geeignete Verbindungen sind beispielsweise Vinylester von gesättigten $C_1$- bis $C_4$-Carbonsäuren wie Vinylformiat, Vinylacetat oder Vinylpropionat, Alkylvinylether mit mindestens 2 C-Atomen in der Alkylgruppe, wie z. B. Ethylvinylether oder Butylvinylether, Ester von monoethylenisch ungesättigten $C_3$- bis $C_6$-Carbonsäuren, z. B. Ester aus einwertigen $C_1$- bis $C_{18}$-Alkoholen und Acrylsäure, Methacrylsäure oder Maleinsäure, Halbester von Maleinsäure, z. B. Maleinsäuremono-methylester, N-Vinyllactame wie N-Vinylpyrrolidon oder N-Vinylcaprolactam, Acrylsäure- und Methacrylsäureester von alkoxylierten einwertigen, gesättigten Alkoholen, z. B. von Alkoholen mit 10 bis 25 C-Atome, die mit 2 bis 200 Mol Ethylenoxid und/oder Propylenoxid pro Mol Alkohol umgesetzt worden sind, sowie Monoacrylsäureester und Monomethacrylsäureester von Polyethylenglykol oder Polypropylenglykol, wobei die Molmassen ($M_n$) der Polyalkylenglykole beispielsweise bis zu 2000 betragen können. Weiterhin geeignete Monomere sind Styrol und alkylsubstituierte Styrole wie Ethylstyrol oder tert.-Butylstyrol.

Diese keine säuregruppentragenden Monomere können auch in Mischung mit anderen Monomeren eingesetzt werden, z. B. Mischungen aus Vinylacetat und 2-Hydroxyethylacrylat in beliebigem Verhältnis. Diese keine Säuregruppen tragenden Monomere werden der Reaktionsmischung in Mengen zwischen 0 und 50 Gew.-%, vorzugsweise kleiner 20 Gew.-% zugesetzt.

Bevorzugt werden vernetzte Polymere aus Säuregruppen tragenden monoethylenisch ungesättigten Monomeren, die gegebenenfalls vor oder nach der Polymerisation in ihre Alkali- oder Ammoniumsalze überführt werden, und aus 0 - 40 Gew.-% bezogen auf ihr Gesamtgewicht keine säuregruppentragenden monoethylenisch ungesättigten Monomeren.

Bevorzugt werden vernetzte Polymere aus monoethylenisch ungesättigten $C_3$- bis $C_{12}$-Carbonsäuren und/oder deren Alkali- oder Ammoniumsalzen. Insbesondere werden vernetzte Polyacrylsäuren bevorzugt, deren Säuregruppen zu 5 bis 30 Mol%, vorzugsweise bei 5 bis 20 Mol%, besonders bevorzugt bei 5 bis 10 Mol%, bezogen auf die Säuregruppen enthaltenden Monomere als Alkali- oder Ammoniumsalze vorliegen.

Als Vernetzer können Verbindungen fungieren, die mindestens zwei ethylenisch ungesättigte Doppelbindungen aufweisen. Beispiele für Verbindungen dieses Typs sind N,N'-Methylenbisacrylamid, Polyethylenglykoldiacrylate und Polyethylenglykoldimethacrylate, die sich jeweils von Polyethylenglykolen eines Molekulargewichts von 106 bis 8500, vorzugsweise 400 bis 2000, ableiten, Trimethylolpropantriacrylat, ethoxyliertes Trimethylolpropantriacrylat (ETMPTA) insbesondere ETMPTA, das mit 15 EO im Mittel ethoxyliert ist, Trimethylolpropantrimethacrylat, Ethylenglykoldiacrylat, Ethylenglykoldimethacrylat, Propylenglykoldiacrylat Propylenglykoldimethacrylat, Butandioldiacrylat, Butandioldimethacrylat, Hexandioldiacrylat, Hexandioldimethacrylat, Allylmethacrylat, Diacrylate und Dimethacrylate von Blockcopolymerisaten aus Ethylenoxid und Propylenoxid, zweifach bzw. mehrfach mit Acrylsäure oder Methacrylsäure veresterte mehrwertige Alkohole, wie Glycerin oder Pentaerythrit, Triallylamin, Dialkyldiallylammoniumhalogenide wie Dimethyldiallylammoniumchlorid und Diethyldiallylammoniumchlorid, Tetraallylethylendiamin, Divinylbenzol, Diallylphthalat, Polyethylenglykoldivinylether von Polyethylenglykolen eines Molekulargewichtes von 106 bis 4000, Trimethylolpropandiallylether, Butandioldivinylether, Pentaerythrittriallylether, Umsetzungsprodukte von 1 Mol Ethylenglykoldiglycidylether oder Polyethylenglykoldiglycidylether mit 2 Mol Pentaerythritoltriallylether oder Allylalkohol, und/oder Divinylethylenharnstoff. Vorzugsweise setzt man wasserlösliche Vernetzer ein, z. B. N,N'-Methylenbisacrylamid, Polyethylenglykoldiacrylate und Polyethylenglykol-dimethacrylate, die sich von Additionsprodukten von 2 bis 400 Mol Ethylenoxid an 1 Mol eines Diols oder Polyols ableiten, Vinylether von Additionsprodukten von 2 bis 400 Mol Ethylenoxid an 1 Mol eines Diols oder Polyols, Ethylenglykoldiacrylat, Ethylenglykoldimethacrylat oder Triacrylate und Trimethacrylate von Additionsprodukten von 6 bis 20 Mol Ethylenoxid an 1 Mol Glycerin, Pentaerythrittriallylether und/oder Divinylharnstoff .

Als Vernetzer kommen außerdem Verbindungen in Betracht, die mindestens eine polymerisierbare ethylenisch ungesättigte Gruppe und mindestens eine weitere funktionelle Gruppe enthalten. Die funktionelle Gruppe dieser Vernetzer muss in der Lage sein, mit den funktionellen Gruppen, im wesentlichen den Säuregruppen, der Monomeren zu reagieren. Geeignete funktionelle Gruppen sind beispielsweise Hydroxyl-, Amino-, Epoxi- und Aziridinogruppen. Verwendung finden können z. B. Hydroxyalkylester der oben genannten monoethylenisch ungesättigten Carbonsäuren, z. B. 2-Hydroxyethylacrylat, Hydroxypropylacrylat, Hydroxybutylacrylat, Hydroxyethylmethacrylat, Hydroxypropylmethacrylat und Hydroxybutylmethacrylat, Allylpiperidiniumbromid, N-Vinylimidazole wie z. B. N-Vinylimidazol, 1-Vinyl-2-methylimidazol und N-Vinylimidazoline wie N-Vinylimidazolin, 1-Vinyl-2-methylimidazolin, 1-Vinyl-2-ethylimidazolin oder 1-Vinyl-2-propylimidazolin, die in Form der freien Basen, in quaternisierter Form oder als Salz bei der Polymerisation eingesetzt werden können. Außerdem eignen sich Dialkylaminoethylacrylat, Dimethylaminoethylmethacrylat, Diethylaminoethylacrylat und Diethylaminoethylmethacrylat. Die basischen Ester werden vorzugsweise in quaternisierter Form oder als Salz eingesetzt. Weiterhin kann z. B. auch Glycidyl(meth)acrylat eingesetzt werden.

Weiterhin kommen als Vernetzer Verbindungen in Betracht, die mindestens zwei funktionelle Gruppen enthalten, die in der Lage sind, mit den funktionellen Gruppen, im wesentlichen den Säuregruppen der Monomeren zu reagieren. Die hierfür geeigneten funktionellen Gruppen wurden bereits oben genannt, d. h. Hydroxyl-, Amino-, Epoxi-, Isocyanat-, Ester-, Amido- und Aziridinogruppen. Beispiele für solche Vernetzer sind Ethylenglykol, Diethylenglykol, Triethylenglykol, Tetraethylenglykol, Polyethylenglykol, Glycerin, Polyglycerin, Triethanolamin, Propylenglykol, Polypropylenglykol, Blockcopolymerisate aus Ethylenoxid und Propylenoxid, Ethanolamin, Sorbitanfettsäureester, ethoxylierte Sorbitanfettsäureester, Trimethylolpropan, Pentaerythrit, 1,3-Butandiol, 1,4-Butandiol, Polyvinylalkohol, Sorbit, Stärke, Polyglycidylether wie Ethylenglykoldiglycidylether, Polyethylenglykol-diglycidylether, Glycerindiglycidylether, Glycerinpolyglyci-dylether, Diglycerinpolyglycidylether, Polyglycerinpolyglycidylether, Sorbitpolyglycidylether, Pentaerythritpolyglycidyle-ther, Propylenglykoldiglycidylether und Polypropylenglykoldiglycidylether, Polyaziridinverbindungen wie 2,2-Bishydroxymethylbutanoltris[3-(1-aziridinyl)-propionat], 1,6-Hexamethylendiethylenharnstoff, Diphenylmethan-bis-4,4'-N,N'-diethylenharnstoff, Halogenepoxyverbindungen wie Epichlorhydrin und ?-Methylepifluorhydrin, Polyisocyanate wie 2,4-Toluylendiisocyanat und Hexamethylendiisocyanat, Alkylencarbonate wie 1,3-Dioxolan-2-on und 4-Methyl-1,3-dioxolan-2-on, weiterhin Bisoxazoline und Oxazolidone, Polyamidoamine sowie deren Umsetzungsprodukte mit Epichlorhydrin, ferner polyquaternäre Amine wie Kondensationsprodukte von Dimethylamin mit Epichlorhydrin, Homo- und Copolymere von Diallyldimethylammoniumchlorid sowie Homo- und Copolymerisate von Dimethylaminoethyl(meth)acrylat, die gegebenenfalls mit beispielsweise Methylchlorid quaterniert sind.

Weitere geeignete Vernetzer sind polyvalente Metallionen, die in der Lage sind, ionische Vernetzungen auszubilden. Beispiele für solche Vernetzer sind Magnesium-, Calcium-, Barium- und Aluminiumionen. Diese Vernetzer werden beispielsweise als Hydroxide, Carbonate oder Hydrogencarbonate eingesetzt. Weitere geeignete Vernetzer sind multifunktionelle Basen, die ebenfalls in der Lage sind, ionische Vernetzungen auszubilden, beispielsweise Polyamine oder deren quaternierte Salze. Beispiele für Polyamine sind Ethylendiamin, Diethylentriamin; Triethylentetramin, Tetraethylenpentamin, Pentaethylenhexamin und Polyethylenimine sowie Polyamine mit Molmassen von jeweils bis zu 4000000.

Die Vernetzer sind in der Reaktionsmischung beispielsweise von 0,001 bis 20 und vorzugsweise von 0,01 bis 14 Gew.-% bezogen auf Monomer vorhanden.

Radikalische Polymerisation

Die Polymerisation wird wie allgemein üblich durch einen Initiator ausgelöst. Auch eine Initiierung der Polymerisation durch Einwirkung von Elektronenstrahlen auf die polymerisierbare, wässrige Mischung ist möglich. Die Polymerisation kann allerdings auch in Abwesenheit von Initiatoren der obengenannten Art durch Einwirkung energiereicher Strahlung in Gegenwart von Photoinitiatoren ausgelöst werden. Als Polymerisationsinitiatoren können sämtliche unter den Polymerisationsbedingungen in Radikale zerfallende Verbindungen eingesetzt werden, z. B. Peroxide, Hydroperoxide, Wasserstoffperoxide, Persulfate, Azoverbindungen und die sogenannten Redoxkatalysatoren. Bevorzugt ist der Einsatz von wasserlöslichen Initiatoren. In manchen Fällen ist es vorteilhaft, Mischungen verschiedener Polymerisationsinitiatoren zu verwenden, z. B. Mischungen aus Wasserstoffperoxid und Natrium- oder Kaliumperoxodisulfat. Mischungen aus Wasserstoffperoxid und Natriumperoxodisulfat können in jedem beliebigen Verhältnis verwendet werden. Geeignete organische Peroxide sind beispielsweise Acetylacetonperoxid, Methylethylketonperoxid, tert.-Butylhydroperoxid, Cumolhydroperoxid, tert.-Amylperpivalat, tert.-Butylperpivalat, tert.-Butylperneohexanoat, tert.-Butylperisobutyrat, tert.-Butyl-per-2-ethylhexanoat, tert.-Butylperisononanoat, tert.-Butylpermaleat, tert.-Butylperbenzoat, Di-(2-ethylhexyl) peroxidicarbonat, Dicyclohexylperoxidicarbonat, Di-(4-tert.-butylcyclohexyl)peroxidicarbonat, Dimyristilperoxidicarbonat, Diacetylperoxidicarbonat, Allylperester, Cumylperoxyneodecanoat, tert.-Butylper-3,5,5-trimethylhexanoat, Acetylcyclohexylsulfonylperoxid, Dilaurylperoxid, Dibenzoylperoxid und tert.-Amylperneodekanoat. Besonders geeignete Polymerisationsinitiatoren sind wasserlösliche Azostarter, z. B. 2,2'-Azo-bis-(2-amidinopropan)dihydrochlorid, 2,2'-Azobis-(N,N'-dimethylen) isobutyramidin-dihydrochlorid, 2-(Carbamoylazo)isobutyronitril, 2,2'-Azobis[2-(2'-imidazolin-2-yl)propan]dihydrochlorid und 4,4'-Azobis-(4-cyanovaleriansäure). Die genannten Polymerisationsinitiatoren werden in üblichen Mengen eingesetzt, z. B. in Mengen von 0,01 bis 5, vorzugsweise 0,05 bis 2,0 Gew.-%, bezogen auf die zu polymerisierenden Monomeren.

Als Initiatoren kommen außerdem Redoxkatalysatoren in Betracht. Die Redoxkatalysatoren enthalten als oxidierende Komponente mindestens eine der oben angegebenen Perverbindungen und als reduzierende Komponente beispielsweise Ascorbinsäure, Glukose, Sorbose, Ammonium- oder Alkalimetallhydrogensulfit, -sulfit, -thiosulfat, -hyposulfit, -pyro-sulfit oder -sulfid, Metallsalze, wie Eisen(II)-ionen oder Natriumhydroxymethylsulfoxylat. Vorzugsweise verwendet man als reduzierende Komponente des Redoxkatalysators Ascorbinsäure oder Natriumsulfit. Bezogen auf die bei der Polymerisation eingesetzte Menge an Monomeren verwendet man beispielsweise $3 \times 10^{-6}$ bis 1 Mol-% der reduzierenden Komponente des Redoxkatalysatorsystems und 0,001 bis 5,0 Mol-% der oxidierenden Komponente des Redoxkatalysators.

Wenn man die Polymerisation durch Einwirkung energiereicher Strahlung auslöst, verwendet man üblicherweise als Initiator sogenannte Photoinitiatoren. Hierbei kann es sich beispielsweise um sogenannte $\alpha$-Spalter, H-abstrahierende Systeme oder auch um Azide handeln. Beispiele für solche Initiatoren sind Benzophenon-Derivate wie Michlers-Keton, Phenanthren-Derivate, Fluoren-Derivate, Anthrachinon-Derivate, Thioxanton-Derivate, Cumarin-Derivate, Benzoinether und deren Derivate, Azoverbindungen wie die oben genannten Radikalbildner, substituierte Hexaarylbisimidazole oder Acylphosphinoxide. Beispiele für Azide sind: 2-(N,N-Dimethylamino)-ethyl-4-azidocinnamat, 2-(N,N-Dimethyl-amino)-ethyl-4-azidonaphthylketon, 2-(N,N-Dimethylamino)-ethyl-4-azidobenzoat, 5-Azido-1-naphthyl-2'-(N,N-dimethylamino)ethylsulfon, N-(4-Sulfonylazidophenyl)mäleinimid, N-Acetyl-4-sulfonylazidoanilin, 4-Sulfonylazidoanilin, 4-Azidoanilin, 4-Azidophenacylbromid, p-Azidobenzoesäure, 2,6-Bis(p-azidobenzyliden)cyclohexanon und 2,6-Bis-(p-azidobenzyliden)-4-methylcyclohexanon. Die Photoinitiatoren werden, falls sie eingesetzt werden, üblicherweise in Mengen von 0,01 bis 5 Gew.-% bezogen auf die zu polymerisierenden Monomeren angewendet.

Die vernetzten Polymere werden vorzugsweise teilneutralisiert eingesetzt. Der Neutralisationsgrad beträgt bevorzugt 5 bis 60 Mol%, vorzugsweise 10 bis 40 Mol%, besonders bevorzugt 20 bis 30 Mol%, bezogen auf die Säuregruppen enthaltenden Monomere. Als Neutralisationsmittel kommen in Frage: Alkalimetallbasen oder Ammoniak bzw. Amine. Vorzugsweise wird Natronlauge, Kalilauge oder Lithiumhydroxid verwendet. Die Neutralisation kann jedoch auch mit Hilfe von Natriumcarbonat, Natriumhydrogencarbonat, Kaliumcarbonat oder Kaliumhydrogencarbonat oder anderen Carbonaten oder Hydrogencarbonaten oder Ammoniak vorgenommen werden. Darüberhinaus sind prim., sek. und tert. Amine einsetzbar.

Alternativ kann die Einstellung des Neutralisationsgrades vor, während oder nach der Polymerisation in allen dafür geeigneten Apparaturen vorgenommen werden. Die Neutralisation kann beispielsweise bei Verwendung eines Kneters zur Polymerisation auch direkt hierin vorgenommen werden. Der verschiedene Neutralisationsgrad bedingt unterschiedliche pH-Werte der Polymerisate.

Als technische Verfahren zur Herstellung dieser Produkte können alle Verfahren Anwendung finden, die üblicherweise bei der Herstellung von Superabsorbern eingesetzt werden, wie sie z. B. im Kapitel 3 in "Modern Superabsorbent Polymer Technology", F.L. Buchholz and A.T. Graham, Wiley-VCH, 1998, erläutert sind.

Bevorzugt ist die Polymerisation in wässriger Lösung als sogenannte Gelpolymerisation. Dabei werden 10 bis 70 Gew.-%ige wässrige Lösungen der Monomere und gegebenenfalls einer geeigneten Pfropfgrundlage in Gegenwart eines Radikalinitiators unter Ausnutzung des Trommsdorff-Norrish-Effektes polymerisiert.

Die Polymerisationsreaktion kann im Temperaturbereich zwischen 0 °C und 150 °C, vorzugsweise zwischen 10 °C und 100 °C, sowohl bei Normaldruck als auch unter erhöhtem oder erniedrigtem Druck durchgeführt werden. Wie üblich kann die Polymerisation auch in einer Schutzgasatmosphäre, vorzugsweise unter Stickstoff, ausgeführt werden.

Durch mehrstündiges Nachheizen der Polymerisatgele im Temperaturbereich 50 bis 130 °C, vorzugsweise 70 bis 100 °C, können die Qualitätseigenschaften der Polymerisate noch verbessert werden.

Oberflächennachvernetzung

Bevorzugt werden Hydrogel-formende Polymere, die oberflächennachvernetzt sind. Die Oberflächennachvernetzung kann in an sich bekannter Weise mit getrockneten, gemahlenen und abgesiebten Polymerpartikeln geschehen.

Hierzu werden Verbindungen, die mit den funktionellen Gruppen der Polymere unter Vernetzung reagieren können, vorzugsweise in Form einer wasserhaltigen Lösung auf die Oberfläche der Hydrogel-Partikel aufgebracht. Die wasserhaltige Lösung kann wassermischbare organische Lösungsmittel enthalten. Geeignete Lösungsmittel sind Alkohole wie Methanol, Ethanol, i-Propanol, Ethylenglycol, Propylenglycol oder Aceton.

Bei der nachträglichen Vernetzung werden Polymere, die durch die Polymerisation der oben genannten monoethylenisch ungesättigten Säuren und gegebenenfalls monoethylenisch ungesättigten Comonomere hergestellt wurden und die ein Molekulargewicht größer 5000, bevorzugt größer 50000 aufweisen, mit Verbindungen umgesetzt, die mindestens zwei gegenüber Säuregruppen reaktive Gruppen aufweisen. Diese Umsetzung kann bei Raumtemperatur oder aber bei erhöhten Temperaturen bis zu 220 °C erfolgen.

Geeignete Nachvernetzungsmittel sind beispielsweise

- Di- oder Polyglycidylverbindungen wie Phosphonsäurediglycidylether oder Ethylenglykoldiglycidylether, Bischlorhydrinether von Polyalkylenglykolen,

- Alkoxysilylverbindungen,
- Polyaziridine, Aziridin-Einheiten enthaltende Verbindungen auf Basis von Polyethern oder substituierten Kohlenwasserstoffen, beispielsweise Bis-N-aziridinomethan,
- Polyamine oder Polyamidoamine sowie deren Umsetzungsprodukte mit Epichlorhydrin,
- Polyole wie Ethylenglykol, 1,2-Propandiol, 1,4-Butandiol, Glycerin, Methyltriglykol, Polyethylenglykole mit einem mittleren Molekulargewicht $M_w$ von 200 - 10000, Di- und Polyglycerin, Pentaerythrit, Sorbit, die Oxethylate dieser Polyole sowie deren Ester mit Carbonsäuren oder der Kohlensäure wie Ethylencarbonat oder Propylencarbonat,
- Kohlensäurederivate wie Harnstoff, Thioharnstoff, Guanidin, Dicyandiamid, 2-Oxazolidinon und dessen Derivate, Bisoxazolin, Polyoxazoline, Di- und Polyisocyanate,
- Di- und Poly-N-methylolverbindungen wie beispielsweise Methylenbis(N-methylol-methacrylamid) oder Melamin-Formaldehyd-Harze,
- Verbindungen mit zwei oder mehr blockierten Isocyanat-Gruppen wie beispielsweise Trimethylhexamethylendiisocyanat blockiert mit 2,2,3,6-Tetramethyl-piperidinon-4.

Bei Bedarf können saure Katalysatoren wie beispielsweise p-Toluolsulfonsäure, Phosphorsäure, Borsäure oder Ammoniumdihydrogenphosphat zugesetzt werden.

Besonders geeignete Nachvernetzungsmittel sind Di- oder Polyglycidylverbindungen wie Ethylenglykoldiglycidylether, die Umsetzungsprodukte von Polyamidoaminen mit Epichlorhydrin und 2-Oxazolidinon.

Das Aufbringen der Vernetzer-Lösung auf das Feinkorn erfolgt bevorzugt durch Aufsprühen einer Lösung des Vernetzers in herkömmlichen Reaktionsmischern oder Misch- und Trocknungsanlagen wie beispielsweise Patterson-Kelly-Mischer, DRAIS-Turbulenzmischer, Lödige-Mischer, Schneckenmischer, Tellermischer, Wirbelschichtmischer und Schugi-Mix. Nach Aufsprühen der Vernetzer-Lösung kann ein Temperaturbehandlungsschritt nachfolgen, bevorzugt in einem nachgeschalteten Trockner, bei einer Temperatur zwischen 80 und 230 °C, bevorzugt 80 - 190 °C, und besonders bevorzugt zwischen 100 und 160 °C, über einen Zeitraum von 5 Minuten bis 6 Stunden, bevorzugt 10 Minuten bis 2 Stunden und besonders bevorzugt 10 Minuten bis 1 Stunde, wobei sowohl Spaltprodukte als auch Lösungsmittelanteile entfernt werden können. Die Trocknung kann aber auch im Mischer selbst erfolgen, durch Beheizung des Mantels oder Einblasen eines vorgewärmten Trägergases.

In einer besonders bevorzugten Ausführung der Erfindung wird zusätzlich die Hydrophilie der Partikeloberfläche der Hydrogel-formenden Polymere durch Ausbildung von Komplexen modifiziert. Die Bildung der Komplexe auf der äußeren Schale der Hydrogel-Partikel erfolgt durch Aufsprühen von Lösungen zwei- oder mehrwertiger Metallsalz-Lösungen, wobei die Metall-Kationen mit den Säuregruppen des Polymers unter Ausbildung von Komplexen reagieren können. Beispiele für zwei- oder mehrwertige Metall-Kationen sind $Mg^{2+}$, $Ca^{2+}$, $Al^{3+}$, $Sc^{3+}$, $Ti^{4+}$, $Mn^{2+}$, $Fe^{2+/3+}$, $Co^{2+}$, $Ni2^+$, $Cu^{+/2+}$, $Zn^{2+}$, $Y^{3+}$, $Zr^{4+}$, $Ag^+$, $La^{3+}$, $Ce^{4+}$, $Hf^{4+}$, und $Au^{+/3+}$, bevorzugte Metall-Kationen sind $Mg^{2+}$, $Ca^{2+}$, $Al^{3+}$, $Ti^{4+}$, $Zr^{4+}$ und $La^{3+}$, und besonders bevorzugte Metall-Kationen sind $Al^{3+}$, $Ti^{4+}$ und $Zr^{4+}$. Die Metall-Kationen können sowohl allein als auch im Gemisch untereinander eingesetzt werden. Von den genannten Metall-Kationen sind alle Metallsalze geeignet, die eine ausreichende Löslichkeit in dem zu verwendenden Lösungsmittel besitzen. Besonders geeignet sind Metallsalze mit schwach komplexierenden Anionen wie zum Beispiel Chlorid, Nitrat und Sulfat. Als Lösungsmittel für die Metallsalze können Wasser, Alkohole, DMF, DMSO sowie Mischungen dieser Komponenten eingesetzt werden. Besonders bevorzugt sind Wasser und Wasser/Alkohol-Mischungen wie zum Bespiel Wasser/Methanol oder Wasser/1,2-Propandiol.

Das Aufsprühen der Metallsalz-Lösung auf die Partikel des Hydrogel-formenden Polymers kann sowohl vor als auch nach der Oberflächennachvernetzung der Partikel erfolgen. In einem besonders bevorzugten Verfahren erfolgt die Aufsprühung der Metallsalz-Lösung im gleichen Schritt mit dem Aufsprühen der Vernetzer-Lösung, wobei beide Lösungen getrennt nacheinander oder gleichzeitig über zwei Düsen aufgesprüht werden, oder Vernetzer- und Metallsalz-Lösung vereint über eine Düse aufgesprüht werden können.

Optional kann noch eine weitere Modifizierung der Hydrogel-formenden Polymere durch Zumischung feinteiliger anorganischer Feststoffe, wie zum Beispiel Silica, Aluminiumoxid, Titandioxid und Eisen(II)-oxid erfolgen, wodurch die Effekte der Oberflächennachbehandlung noch weiter verstärkt werden. Besonders bevorzugt ist die Zumischung von hydrophilem Silica oder von Aluminiumoxid mit einer mittleren Größe der Primärteilchen von 4 bis 50 nm und einer spezifischen Oberfläche von 50 - 450 m$^2$/g. Die Zumischung feinteiliger anorganischer Feststoffe erfolgt bevorzugt nach der Oberflächenmodifizierung durch Vernetzung/Komplexbildung, kann aber auch vor oder während diesen Oberflächenmodifizierungen durchgeführt werden.

Das oberflächennachvernetzte Material wird im Allgemeinen getempert.

Temperung, Mantel-Temperatur: 120-180 °C, bevorzugt 140-160 °C, insbesondere 150 °C; Temperung, Verweilzeit muss an die Temperatur angepasst werden, wobei bei höheren Temperaturen kürzere Verweilzeiten vorliegen und bei längeren Verweilzeiten stärkere Nachvernetzung vorliegt. Typische Werte sind 150-10 Minuten.

Durch die Nachvernetzungszeit können die AUL und CRC optimiert werden.

Copolymere aus $C_2$ - $C_8$ Olefinen oder Styrolen mit Anhydriden

Copolymere aus $C_2$ - $C_8$ Olefinen oder Styrolen mit Anhydriden sind bekannt und kommerziell erhältlich. Ihre Herstellung ist ausführlich beschrieben z.B. in US 5066742 und US 5026784, deren Herstellvorschrift hiermit Bestandteil der vorliegenden Erfindung ist.

Eigenschaften der erfindungsgemäßen Polymermischungen

Die wässrige Flüssigkeiten absorbierenden Hydrogel-formenden Polymere weisen im Allgemeinen eine Teilchengrößenverteilung von 10 μm bis etwa 1000 μm, bevorzugt etwa 100 μm bis etwa 850 μm, insbesondere 150 μm bis etwa 700 μm auf. In dem genannten Größenfenster liegen bevorzugt mehr als 80 Gew.-%, insbesondere mehr wie 90 Gew.-% der Teilchen.

Die geruchsbindenden Copolymere aus $C_2$ - $C_8$ Olefinen oder Styrolen mit Anhydriden weisen im Allgemeinen eine Teilchengrößenverteilung von 10 μm bis etwa 600 μm, bevorzugt etwa 100 μm bis etwa 400 μm, insbesondere 150 μm bis etwa 300 μm auf. In dem genannten Größenfenster liegen bevorzugt mehr als 80 Gew.-%, insbesondere mehr wie 90 Gew.-% der Teilchen.

Die wässrige Flüssigkeiten absorbierenden $C_2$ - $C_8$ Olefin / Anhydrid-, insbesondere Maleinsäureanhydrid-Copolymer-Fasern werden vorzugsweise erhalten nach der Methode gemäß US 5,026,784 Example 1 Spalte 8 Zeile 24 und weisen die dort beschriebenen Eigenschaften auf (Neutralisationsgrad 55%, Durchmesser der unvernetzten Faser 2-3 Denier.

Die Polymerenmischungen weisen bei hohem Endabsorptionsvermögen, hoher Gelfestigkeit und Permeabilität, sowie hoher Retention verbesserte Geruchsbindungseigenschaften auf. Durch das Vorliegen von Copolymeren aus $C_2$ - $C_8$ Olefinen oder Styrolen mit Anhydriden weisen die erfindungsgemäßen Produkte antimikrobielle Eigenschaften auf, so dass damit ein Geruchskontrollsystem zugrunde liegt, ohne dass es des Zusatzes weiterer geruchsinhibierender Substanzen oder geruchsmaskierender Materialien bedarf.

Der Zusatz teilneutralisierter Copolymere aus $C_2$ - $C_8$ Olefinen oder Styrolen mit Anhydriden in Faserform ermöglicht höhere Akquisitionsraten sowie höhere Retentionswerte, als es bei der erfindungsgemäßen granulären Polymermischung der Fall ist.

Im Gegensatz zum Stand der Technik, bei dem eine zugesetzte Geruchskontrolleinheit zu den hochquellfähigen Polymeren zum Abfall an Absorptionsleistung führt, kommt es bei der erfindungsgemäßen Polymerenmischung zu keiner negativen Beeinflussung des Absorptionsprofils. Darüber hinaus kann bei den erfindungsgemäßen Produkten eine weitaus kostengünstigere Herstellung erfolgen, da auf Bindemittel oder sonstige Anbindungshilfen von Geruchskontrolleinheit an Hydrogel-formende Polymere verzichtet werden kann.

Die hohe Absorptionsleistung und ein unverändert Absorptionsverhalten der eingesetzten Hydrogel-formenden Polymeren ermöglicht längere Tragezeiten bei Einsatz der erfindungsgemäßen Produkte im Hygieneartikel. Hautreizungen und Hautirritationen werden durch ein gleichbleibendes pH-Milieu vollständig vermieden und ausgeschlossen.

Einsatz und Verwendung der Polymermischung

Die vorliegende Erfindung betrifft ferner die Verwendung der oben genannten Polymerenmischung in Hygieneartikel, umfassend

(A) eine obere flüssigkeitsdurchlässige Abdeckung
(B) eine untere flüssigkeitsundurchlässige Schicht
(C) einen zwischen (A) und (B) befindlichen Kern, enthaltend
10 - 100 Gew.-% des erfindungsgemäßem Polymerenmischung
0 - 90 Gew.-% hydrophiles Fasermaterial
bevorzugt 20 - 100 Gew.-% des erfindungsgemäßem Polymerenmischung, 0 - 80 Gew.-% hydrophiles Fasermaterial
mehr bevorzugt 30 - 100 Gew.-% des erfindungsgemäßem Polymerenmischung, 0 - 70 Gew.-% hydrophiles Fasermaterial
noch mehr bevorzugt 40 - 100 Gew.-% des erfindungsgemäßem Polymerenmischung, 0 - 60 Gew.-% hydrophiles Fasermaterial besser bevorzugt 50 - 100 Gew.-% des erfindungsgemäßem Polymerenmischung 0 - 50 Gew.-% hydrophiles Fasermaterial besonders bevorzugt 60 - 100 Gew.-% des erfindungsgemäßem Polymerenmischung, 0 - 40 Gew.-% hydrophiles Fasermaterial insbesondere bevorzugt 70 - 100 Gew.-% des erfindungsgemäßem Polymerenmischung, 0 - 30 Gew.-% hydrophiles Fasermaterial außerordentlich bevorzugt 80 - 100 Gew.-% des erfindungsgemäßem Polymerenmischung, 0 - 20 Gew.-% hydrophiles Fasermaterial
am meisten bevorzugt 90 - 100 Gew.-% des erfindungsgemäßem Polymerenmischung, 0 - 10 Gew.-% hydrophiles Fasermaterial

(D) gegebenenfalls eine sich unmittelbar oberhalb und unterhalb des Kerns (C) befindende Tissueschicht und

(E) gegebenenfalls eine zwischen (A) und (C) sich befindende Aufnahmeschicht.

Das hydrophile Fasermaterial kann ganz oder teilweise durch Fasermaterial aus Copolymeren aus $C_2$ - $C_8$ Olefinen oder Styrolen mit Anhydriden ersetzt sein. Die Prozentangaben sind so zu verstehen, dass bei 10 - 100 Gew. %, 11, 12, 13, 14, 15, 16, 17, 18, 19 bis jeweils 100 Gew.-% an erfindungsgemäßem Polymerenmischung und alle dazwischenliegenden %-Angaben (z.B. 12,2%) möglich sind und entsprechend hydrophiles Fasermaterial von 0 bis jeweils 89, 88, 87, 86, 85, 83, 82, 81 Gew.-% und dazwischen liegende Prozentangaben (z.B. 87,8%) möglich sind. Liegen weitere Materialien im Kern vor, so verringern sich entsprechend die Prozentwerte an Polymer und Faser. Das ananloge gilt für die bevorzugten Bereiche, so z.B. außerordentlich bevorzugt können 81, 82, 83, 84, 85, 86, 87, 88, 89 Gew.% für das erfindungsgemäßem Polymerenmischung und entsprechend 19, 18, 17, 16, 15, 14, 13, 12, 11 Gew-% des Fasermaterials vorliegen. So können im bevorzugten Bereich 20, 21, 22, 23, 24, 25, 26, 27, 28, 29 bis 100 Gew.-% erfindungsgemäßes Polymerenmischung, im mehr bevorzugten Bereich 30, 31, 32, 33, 34, 35, 36, 37, 38, 39 bis 100 Gew.-% erfindungsgemäßes Polymerenmischung, im noch mehr bevorzugten Bereich 40, 41, 42, 43, 44, 45, 46, 47, 48, 49 bis 100 Gew.-% erfindungsgemäßes Polymerenmischung, im besser bevorzugten Bereich 50, 51, 52, 53, 54, 55, 56, 57, 58, 59 bis 100 Gew.-% erfindungsgemäßes Polymerenmischung, im besonders bevorzugten Bereich 60, 61, 62, 63, 64, 65, 66, 67, 68, 69 bis 100 Gew.-% erfindungsgemäßes Polymerenmischung, im insbesonders bevorzugten Bereich 70, 71, 71, 72, 73, 74, 75, 76, 77, 78, 79 bis 100 Gew.-% erfindungsgemäßes Polymerenmischung und im am meisten bevorzugten Bereich 90, 91, 92, 93, 94, 95, 96, 97, 98, 99 oder 100 Gew.-% erfindungsgemäßes Polymerenmischung vorliegen.

Unter Hygieneartikel sind dabei sowohl Inkontinenzeinlagen und Inkontinenzhosen für Erwachsene als auch Windeln für Babys zu verstehen.

Bei der flüssigkeitsdurchlässigen Abdeckung (A) handelt es sich um die Schicht, die direkten Hautkontakt hat. Das Material hierfür besteht hierbei aus üblichen synthetischen oder halbsynthetischen Fasern oder Filme von Polyester, Polyolefine, Rayon oder natürlichen Fasern wie Baumwolle. Bei nichtgewebten Materialien sind die Fasern in der Regel durch Bindemittel wie Polyacrylate zu verbinden. Bevorzugte Materialien sind Polyester, Rayon und deren Blends, Polyethylen und Polypropylen. Beispiele für flüssigkeitsdurchlässige Schichten sind beschrieben in WO 99/57355 A1, EP 102 388 3 A2.

Die flüssigkeitsundurchlässige Schicht (B) besteht in der Regel aus einer Folie aus Polyethylen oder Polypropylen.

Der Kern (C) enthält neben der erfindungsgemäßen Polymerenmischung hydrophiles Fasermaterial. Unter hydrophil ist zu verstehen, dass sich wässrige Flüssigkeiten schnell über die Faser verteilen. Für gewöhnlich ist das Fasermaterial Cellulose, modifizierte Cellulose, Rayon, Polyester wie Polyethylenterephthalat. Besonders bevorzugt werden Cellulosefasern wie Zellstoff. Die Fasern haben in der Regel einen Durchmesser von 1 - 200 $\mu$m, bevorzugt 10 - 100 $\mu$m. Darüberhinaus haben die Fasern eine Mindestlänge von 1 mm.

Der Aufbau und die Form von Windeln ist allgemein bekannt und beispielsweise in der WO 95 / 26 209 S. 66 Zeile 34 bis S. 69 Zeile 11, DE 196 04 601 A1, EP-A-0 316 518 und EP-A-0 202 127 beschrieben. Allgemein werden Windeln und andere Hygieneartikel auch in WO 00/65084, insbesondere auf Seiten 6-15, WO 00/65348, insbesondere auf Seiten 4 - 17, WO 00/35502, insbesondere Seiten 3-9, DE 19737434, WO 98/8439 beschrieben. Hygieneartikel für die Damenhygiene werden in folgenden Literaturstellen beschrieben. Die erfindungsgemäßen wässrige Flüssigkeiten Polymerenmischungen können dort eingestzt werden. Literaturstellen Damenhygiene: WO 95/24173: Absorption Article for Controlling Odour, WO 91/11977: Body Fluid Odour Control, EP 389023: Absorbent Sanitary Articles, WO 94/25077: Odour Control Material, WO 97/01317: Absorbent Hygienic Article, WO 99/18905, EP 834297, US 5,762,644, US 5,895,381, WO 98/57609, WO 2000/065083, WO 2000/069485, WO 2000/069484, WO 2000/069481, US 6,123,693, EP 1104666, WO 2001/024755, WO 2001/000115, EP 105373, WO 2001/041692, EP 1074233. Tampons werden in folgenden Schriften beschrieben: WO 98/48753, WO 98/41179, WO 97/09022, WO 98/46182, WO 98/46181, WO 2001/043679, WO 2001/043680, WO 2000/061052, EP 1108408, WO 2001/033962, DE 200020662, WO 2001/001910, WO 2001/001908, WO 2001/001909, WO 2001/001906, WO 2001/001905, WO 2001/24729. Inkontinenzartikel werden in folgenden Schriften beschrieben: Disposable Absorbent Article for Incontinent Individuals: EP 311344 Beschreibung S. 3 - 9; Disposable Absorbent Article: EP 850623; Absorbent Article: WO 95/26207; Absorbent Article: EP 894502; Dry Laid Fibrous Structure: EP 850 616; WO 98/22063; WO 97/49365; EP 903134; EP 887060; EP 887059; EP 887058; EP 887057; EP 887056; EP 931530; WO 99/25284; WO 98/48753. Damenhygiene- und Inkontinenzartikel wird in folgenden Schriften beschrieben: Catamenial Device: WO 93/22998 Beschreibung S. 26 - 33; Absorbent Members for Body Fluids: WO 95/26209 Beschreibung S. 36 - 69; Disposable Absorbent Article: WO 98/20916 Beschreibung S. 13 - 24; Improved Composite Absorbent Structures: EP 306262 Beschreibung S. 3 - 14; Body Waste Absorbent Article: WO 99/45973.

Alternativ kann der Kern (C) auch aus Schichten der Komponenten (i) und (ii) bestehen. Es können prinzipiell Mehrschicht-Aufbauten aus 3 (z.B. Schicht aus Komponte (i)/ Schicht aus Komponente (ii)/ Schicht aus Komponente (i) oder Schicht aus Komponte (ii)/ Schicht aus Komponente (i)/ Schicht aus Komponente (ii)), 4, 5 oder mehr Schichten vorliegen, bevorzugt sind aber Aufbauten mit zwei Schichten wobei sowohl die Schicht mit der Komponente (i) als auch mit der Komponente (ii) näher am Körper liegen kann. Möglich sind auch Schichtaufbauten aus Schichten mit erfindungsgemäßen Polymerenmischungen und Schichten aus einzelnen Komponenten (i) und/oder (ii).

Die erfindungsgemäßen Polymerenmischungen eignen sich hervorragend als Absorptionsmittel für Wasser und wässrige Flüssigkeiten, so dass sie vorteilhaft als Wasser zurückhaltendes Mittel im landwirtschaftlichen Gartenbau, als Filtrationshilfsmittel und besonders als saugfähige Komponente in Hygieneartikeln wie Windeln, Tampons oder Damenbinden eingesetzt werden können.

Einlagerung und Fixierung der erfindungsgemäßen hochquellfähigen Polymermischungen

Zusätzlich zu der oben beschriebenen Polymerenmischung aus Hydrogel-bildenden Polymerisaten und Copolymeren aus $C_2$ - $C_8$ Olefinen oder Styrolen mit Anhydriden können in der absorbierenden Zusammensetzung gemäß der vorliegenden Erfindung Kompositionen vorliegen, welche die Polymerenmischung enthält oder an denen sie fixiert ist. Jede Komposition ist geeignet, die die Polymerenmischung aufnehmen kann und die darüber hinaus in die Absorptionsschicht integriert werden kann. Eine Vielzahl derartiger Zusammensetzungen ist bereits bekannt und eingehend in der Literatur beschrieben. Eine Komposition zum Einbau der Polymerenmischung kann z. B. eine Fasermatrix sein, die aus einem Cellulosefasergemisch (airlaid web, wet laid web) oder aus synthetischen Polymerfasern (meltblown web, spunbonded web), oder aber aus einem Misch-Faserwerk aus Cellulosefasern und synthetischen Fasern besteht. Mögliche Fasermaterialien werden im sich anschließenden Kapitel detailliert beschrieben. Der Prozess eines air-laid web ist beispielsweise geschildert in WO 98/28 478. Des weiteren können offenporige Schäume oder ähnliches zum Einbau der erfindungsgemäßen Polymerenmischung dienen.

Alternativ kann eine derartige Komposition durch Fusion zweier Einzelschichten entstehen, wobei eine oder besser eine Vielzahl an Kammern gebildet werden, die die Polymerenmischung enthalten. Ein derartiges Kammersystem ist detailliert geschildert in EP 0 615 736 A1 S. 7 Zeile 26 ff.

In diesem Fall sollte mindestens eine der beiden Schichten wasserdurchlässig sein. Die zweite Schicht kann entweder wasserdurchlässig oder wasserundurchlässig sein. Als Schichtenmaterial können Tissues oder sonstiges Gewebe, geschlossene oder offenporige Schäume, perforierte Filme, Elastomere oder Gewebe aus Fasermaterial zum Einsatz gelangen. Wenn die absorbierende Zusammensetzung aus einer Komposition von Schichten besteht, sollte das Schichtenmaterial eine Porenstruktur aufweisen, deren Porenabmessungen klein genug sind, um die Partikel der Polymerenmischung zurückzuhalten. Obige Beispiele zur Komposition der absorbierenden Zusammensetzung schließen auch Laminate aus mindestens zwei Schichten mit ein, zwischen die die Polymerenmischung eingebaut und fixiert wird.

Generell besteht die Möglichkeit, die Partikel der Polymerenmischung innerhalb des Absorbent Cores zur Verbesserung der sog. Dry- und Wet-Integrity zu fixieren. Unter Dry- und Wet-Integrity versteht man die Fähigkeit, hochquellfähige Hydrogele derart in die absorbierende Zusammensetzung einzubauen, dass sie äußeren Krafteinwirkungen sowohl im nassen als auch im trockenen Zustand standhalten und es nicht zu Verschiebungen oder Austritt von hochquellfähigem Polymer kommt. Unter Krafteinwirkungen sind vor allem mechanische Belastungen zu verstehen, wie sie im Bewegungsablauf beim Tragen des Hygieneartikels auftreten, oder aber die Gewichtsbelastung, unter der der Hygieneartikel vor allem im Inkontinenzfall steht. Zur Fixierung gibt es eine Vielzahl an Möglichkeiten, die dem Fachmann bekannt sind. Beispiele wie die Fixierung durch Wärmebehandlung, Zusatz von Adhäsiven, Thermoplasten, Bindermaterialien sind verzeichnet in WO 95/26 209 S. 37 Zeile 36 bis S. 41 Zeile 14. Methoden zur Erhöhung der Wet Strength finden sich auch in WO 2000/36216 A1.

Des weiteren kann die absorbierende Zusammensetzung aus einem Trägermaterial, wie z. B. einem Polymerfilm bestehen, auf dem die Polymerenmischung fixiert wird. Die Fixierung kann sowohl einals auch beidseitig vorgenommen werden. Das Trägermaterial kann wasserdurchlässig oder wasserundurchlässig sein.

In obige Kompositionen der absorbierenden Zusammensetzung werden Partikel der Polymerenmischung mit einem Gewichtsanteil von 10 bis 100 Gew%, bevorzugt 20 - 100 Gew.-%, mehr bevorzugt 30 - 100 Gew.-%, noch mehr bevorzugt 40 - 100 Gew.-%, besser bevorzugt 50 - 100 Gew.-%, besonders bevorzugt 60 - 100 Gew.-%, insbesondere bevorzugt 70 - 100 Gew.-%, außerordentlich bevorzugt 80 - 100 Gew.-% und am meisten bevorzugt 90 - 100 Gew.-% basierend auf dem Gesamtgewicht der Komposition und der Polymerenmischung eingebaut.

Fasermaterialien der absorbierenden Zusammensetzung

Dem Aufbau der vorliegenden erfindungsgemäßen absorbierenden Zusammensetzung liegen vielfältige Fasermaterialien zugrunde, die als Fasernetzwerk oder Matrices zum Einsatz gelangen. Mit eingeschlossen von der vorliegenden Erfindung sind sowohl Fasern natürlichen Ursprungs (modifiziert oder unmodifiziert), als auch Synthesefasern.

Einen detaillierten Überblick über Beispiele von Fasern, die in der vorliegenden Erfindung eingesetzt werden können, gibt Patent WO 95/26 209 S. 28 Zeile 9 bis S. 36 Zeile 8.

Beispiele für Cellulosefasern schließen jene ein, die üblicherweise bei Absorptionsprodukten verwendet werden, wie Flauschzellstoff und Zellstoff vom Baumwolltyp. Die Materialien (Nadel- oder Laubhölzer), Herstellungsverfahren, wie chemischer Zellstoff, halbchemischer Zellstoff, chemothermischer mechanischer Zellstoff (CTMP) und Bleichverfahren sind nicht besonders eingeschränkt. So finden beispielsweise natürliche Cellulosefasern wie Baumwolle, Flachs, Seide, Wolle, Jute, Ethylcellulose und Celluloseacetat Anwendung.

Geeignete synthetische Fasern werden hergestellt aus Polyvinylchlorid, Polyvinylflourid, Polytetraflourethylen, Polyvinylidenchlorid, Polyacrylverbindungen wie ORLON® ; Polyvinylacetat, Polyethylvinylacetat, löslicher oder unlöslicher Polyvinylalkohol. Beispiele für synthetische Fasern schließen thermoplastische Polyolefinfasern, wie Polyethylenfasern (PULPEX® ), Polypropylenfasern und Polyethylen-Polypropylen-Zweikomponentenfasern, Polyesterfasern, wie Polyethylenterephthalatfasern (DACRON® oder KO-DEL® ), Copolyester, Polyvinylacetat, Polyethylvinylacetat, Polyvinylchlorid, Polyvinylidenchlorid, Polyacryle, Polyamide, Copolyamide, Polystyrol und Copolymere der vorstehend genannten Polymere, sowie Zweikomponentenfasern aus Polyethylenterephthalat-Polyethylen-Isophthalat-Copolymer, Polyethylvinylacetat/Polypropylen, Polyethylen/Polyester, Polypropylen/Polyester, Copolyester/Polyester, Polyamidfasern (Nylon), Polyurethanfasern, Polystyrolfasern und Polyacrylnitrilfasern ein. Bevorzugt sind Polyolefinfasern, Polyesterfasern und deren Zweikomponentenfasern. Weiterhin bevorzugt sind in der Wärme haftende Zweikomponentenfasern aus Polyolefin vom Hülle-Kern-Typ und Seite-an-Seite-Typ wegen ihrer ausgezeichneten Formbeständigkeit nach der Flüssigkeitsabsorption.

Die genannten synthetischen Fasern werden bevorzugt in Kombination mit thermoplastischen Fasern eingesetzt. Bei der Hitzebehandlung migrieren letztere teilweise in die Matrix des vorhandenen Fasermaterials und stellen so beim Abkühlen Verbindungsstellen und erneute Versteifungselemente dar. Zusätzlich bedeutet der Zusatz thermoplastischer Fasern eine Erweiterung der vorliegenden Porenabmessungen nach erfolgter Hitzebehandlung. Auf diese Weise ist es möglich, durch kontinuierliches Zudosieren von thermoplastischen Fasern während der Bildung der Absorptionsschicht den Anteil thermoplastischer Fasern zum Deckblatt hin kontinuierlich zu steigern, wodurch ein ebenso kontinuierlicher Anstieg der Porengrößen resultiert. Thermoplastische Fasern können aus einer Vielzahl thermoplastischer Polymere gebildet werden, die einen Schmelzpunkt von weniger als 190°C, bevorzugt zwischen 75°C und 175°C aufweisen. Bei diesen Temperaturen ist noch keine Schädigung der Cellulosefasern zu erwarten.

Längen und Durchmesser der vorstehend beschriebenen Synthesefasern sind nicht besonders eingeschränkt und im allgemeinen kann jede beliebige Faser mit einer Länge von 1 bis 200 mm und einem Durchmesser von 0.1 bis 100 Denier (Gramm pro 9 000 Meter) bevorzugt verwendet werden. Bevorzugte thermoplastische Fasern weisen eine Länge von 3 bis 50 mm, besonders bevorzugt eine Länge von 6 bis 12 mm auf. Der bevorzugte Durchmesser der thermoplastischen Faser liegt zwischen 1,4 und 10 Decitex, besonders bevorzugt zwischen 1,7 und 3,3 Decitex (Gramm pro 10 000 Meter). Die Form ist nicht besonders eingeschränkt und Beispiele schließen gewebeartige, schmale zylinderartige, geschnitten-/spaltgarnartige, stapelfaserartige und endlosfaserartige ein.

Die Fasern in der erfindungsgemäßen absorbierenden Zusammensetzung können hydrophil, hydrophob oder eine Kombination aus beiden sein. Gemäß der Definition von Robert F. Gould in der Publikation "Kontaktwinkel, Benetzbarkeit und Adhäsion", American Chemical Society (1964) wird eine Faser als hydrophil bezeichnet, wenn der Kontaktwinkel zwischen der Flüssigkeit und der Faser (bzw. ihrer Oberfläche) kleiner als 90º ist, oder wenn die Flüssigkeit zum spontanen Spreiten auf derselben Oberfläche tendiert. Beide Vorgänge sind in aller Regel coexistent. Umgekehrt wird eine Faser als hydrophob bezeichnet, wenn ein Kontaktwinkel von größer als 90° ausgebildet wird und kein Spreiten beobachtet wird.

Bevorzugt wird hydrophiles Fasermaterial eingesetzt. Besonders bevorzugt gelangt Fasermaterial zum Einsatz, das zur Körperseite hin schwach hydrophil und in der Region um die Polymerenmischung am stärksten hydrophil ist. Im Herstellungsprozeß wird durch den Einsatz von Schichten unterschiedlicher Hydrophilie ein Gradient erzeugt, der die auftreffende Flüssigkeit zum Hydrogel kanalisiert, wo letztendlich die Absorption erfolgt.

Geeignete hydrophile Fasern für den Einsatz in der erfindungsgemäßen absorbierenden Zusammensetzung sind beispielsweise Cellulosefasern, modifizierte Cellulosefasern, Rayon, Polyesterfasern wie z. B. Polyethylenterephthalat (DACRON® ), und hydrophiles Nylon (HYDROFIL® ). Geeignete hydrophile Fasern können auch erhalten werden durch Hydrophilierung hydrophober Fasern, wie z.B. die Behandlung thermoplastischer Fasern, erhalten aus Polyolefinen (wie z. B. Polyethylen oder Polypropylen, Polyamide, Polystyrole, Polyurethane usw.) mit Tensiden oder Silica. Aus Kostengründen und aus Gründen der Verfügbarkeit werden jedoch Cellulosefasern bevorzugt.

Die Polymerenmischung wird in das beschriebene Fasermaterial eingebettet. Dies kann auf vielfältige Weise geschehen, indem man z. B. mit dem Polymerenmaterial und den Fasern zusammen eine Absorptionsschicht in Form einer Matrix aufbaut, oder durch Einlagerung der der Polymerpartikelmischung in Schichten aus Fasergemisch, wo sie letztendlich fixiert werden, sei es durch Haftmittel oder Laminierung der Schichten.

Die flüssigkeitsaufnehmende und -verteilende Fasermatrix kann dabei aus synthetischer Faser oder Cellulosefaser oder einem Gemisch aus synthetischer Faser und Cellulosefaser bestehen, wobei das Mischungsverhältnis von (100 bis 0) synthetische Faser : (0 bis 100) Cellulosefaser variieren kann. Die eingesetzten Cellulosefasern können zur Erhöhung der Formbeständigkeit des Hygieneartikels zusätzlich chemisch versteift sein.

Die chemische Versteifung von Cellulosefasern kann auf unterschiedlichen Wegen erreicht werden. Zum einen kann eine Faserversteifung erreicht werden durch Zusatz geeigneter Überzüge / Coatings zum Fasermaterial. Derartige Zusätze schließen beispielsweise Polyamid-Epichlorhydrin-Überzüge (Kymene® 557 H, Hercoles, Inc. Wilmington Delaware, USA), Polyacrylamid- Überzüge (beschrieben in U.S. Patent 3,556,932 oder als Handelsprodukt der Marke Parez® 631 NC, American Cyanamid Co., Stamford, CT, USA), Melamin-Formaldehyd-Überzüge und Polyethylenimin-Überzüge mit ein.

Die chemische Versteifung von Cellulosefasern kann auch durch chemische Reaktion erfolgen. So kann z. B. die Zugabe von geeigneten Vernetzersubstanzen eine Vernetzung bewirken, die innerhalb der Faser stattfindet. Geeignete Vernetzersubstanzen sind typische Substanzen, die zur Vernetzung von Monomeren eingesetzt werden. Mit eingeschlossen, jedoch nicht limitiert darauf, sind $C_2$-$C_8$ Dialdehyde, $C_2$-$C_8$ Monoaldehyde mit saurer Funktionalität, und insbesondere $C_2$-$C_9$ Polycarbonsäuren. Spezifische Substanzen aus dieser Reihe sind beispielsweise Glutaraldehyd, Glyoxal, Glyoxylsäure, Formaldehyd und Citronensäure. Diese Substanzen reagieren mit mindestens 2 Hydroxyl-Gruppen innerhalb einer einzelnen Cellulosekette oder zwischen zwei benachbarten Celluloseketten innerhalb einer einzelnen Cellulosefaser. Durch die Vernetzung erfolgt eine Verteifung der Fasern, die durch diese Behandlung eine größere Formbeständigkeit verliehen bekommen. Zusätzlich zu ihrem hydrophilen Charakter weisen diese Fasern einheitliche Kombinationen aus Versteifung und Elastizität auf. Diese physikalische Eigenschaft ermöglicht es, die kapillare Struktur auch bei gleichzeitigem Kontakt mit Flüssigkeit und Kompressionskräften beizubehalten und ein vorzeitiges Kollabieren zu verhindern.

Chemisch vernetzte Cellulosefaseren sind bekannt und in WO 91/11162, U.S. Patent 3,224,926, U.S. Patent 3,440,135, U.S. Patent 3,932,209, U.S. Patent 4,035,147, U.S. Patent 4,822,453, U.S. Patent 4, 888, 093, U.S. Patent 4,898,642 und U.S. Patent 5,137,537 beschrieben. Die chemische Vernetzung bewirkt eine Versteifung des Fasermaterials, was sich letztendlich in einer verbesserten Formbeständigkeit des gesamten Hygieneartikels widerspiegelt. Die einzelnen Schichten werden durch dem Fachmann bekannte Methoden, wie z. B. Verschmelzen durch Wärmebehandlung, Zugabe von Schmelzklebern, Latexbindern usw. miteinander verbunden.

Herstellungsverfahren der absorbierenden Zusammensetzung

Die absorbierende Zusammensetzung setzt sich zusammen aus Kompositionen, welche die Polymerenmischung enthalten, und der Polymerenmischung, die in besagten Kompositionen vorliegt oder daran fixiert ist.

Beispiele für Verfahren, mit denen man eine absorbierende Zusammensetzung erhält, die beispielsweise aus einem Trägermaterial bestehen, an den ein- oder beidseitig Partikel der Polymerenmischung fixiert sind, sind bekannt und von der Erfindung mit eingeschlossen, jedoch nicht limitiert darauf.

Beispiele für Verfahren, mit denen man eine absorbierende Zusammensetzung erhält, die beispielsweise aus in ein Fasermaterial-Gemisch aus synthetischen Fasern (a) und Cellulosefasern (b) eingebetteter Polymerenmischung (c) besteht, wobei das Mischungsverhältnis von (100 bis 0) synthetische Faser : (0 bis 100) Cellulosefaser variieren kann, schließen (1) ein Verfahren, bei dem (a), (b) und (c) gleichzeitig gemischt werden, (2) ein Verfahren, bei dem ein Gemisch aus (a) und (b) in (c) eingemischt wird, (3) ein Verfahren, bei dem ein Gemisch aus (b) und (c) mit (a) gemischt wird, (4) ein Verfahren, bei dem ein Gemisch aus (a) und (c) in (b) eingemischt wird, (5) ein Verfahren, bei dem (b) und (c) gemischt werden und (a) kontinuierlich zudosiert wird, (6) ein Verfahren, bei dem (a) und (c) gemischt werden und (b) kontinuierlich zudosiert wird, und (7) ein Verfahren, bei dem (b) und (c) getrennt in (a) eingemischt werden, ein. Von diesen Beispielen sind die Verfahren (1) und (5) bevorzugt. Die Vorrichtung, die in diesem Verfahren verwendet wird, ist nicht besonders eingeschränkt und es kann eine übliche, dem Fachmann bekannte Vorrichtung verwendet werden.

Die entsprechend erzeugte absorbierende Zusammensetzung kann optional einer Hitzebehandlung unterworfen werden, so dass eine Absorptionsschicht mit hervorragender Formbeständigkeit im feuchten Zustand resultiert. Das Verfahren zur Hitzebehandlung ist nicht besonders eingeschränkt. Beispiele schließen Hitzebehandlung durch Zufuhr heißer Luft oder Infrarotbestrahlung mit ein. Die Temperatur bei der Hitzebehandlung liegt im Bereich 60°C bis 230°C, bevorzugt zwischen 100°C und 200°C, besonders bevorzugt zwischen 100°C und 180°C.

Die Dauer der Hitzebehandlung hängt ab von der Art der synthetischen Faser, deren Menge und der Herstellungsgeschwindigkeit des Hygieneartikels. Generell beträgt die Dauer der Hitzebehandlung zwischen 0.5 Sekunde bis 3 Minuten, bevorzugt 1 Sekunde bis 1 Minute.

Die absorbierende Zusammensetzung wird im allgemeinen beispielsweise mit einer für Flüssigkeit durchlässigen Deckschicht und einer für Flüssigkeit undurchlässigen Unterschicht versehen. Weiterhin werden Beinabschlüsse und Klebebänder angebracht und so der Hygieneartikel fertiggestellt. Die Materialien und Arten der durchlässigen Deckschicht und undurchlässigen Unterschicht, sowie der Beinabschlüsse und Klebebänder sind dem Fachmann bekannt und nicht besonders eingeschränkt. Beispiele hierfür finden sich in WO 95/26 209.

Testmethoden

a) Zentrifugenretentionskapazität (CRC Centrifuge Retention Capacity)

Bei dieser Methode wird die freie Quellbarkeit der Polymerenmischung im Teebeutel bestimmt. Zur Bestimmung der CRC werden 0.2000 ± 0.0050 g der erfindungsgemäßen Polymergemisches, z. B. trockenen Polymerenmischung, bestehend aus 0.18 g Hydrogel-formendem Polymer (Kornfraktion 106 - 850 $\mu$m) und 0.02 g Copolymere aus $C_2$ - $C_8$ Olefinen oder Styrolen mit Anhydriden (Kornfraktion 100 - 400 $\mu$m) in einem 60 x 85 mm großen Teebeutel eingewogen, der anschließend verschweißt wird. Der Teebeutel wird für 30 Minuten in einen Überschschuß von 0.9 Gew.%igen Kochsalzlösung gegeben (mindestens 0,83 l Kochsalz-Lösung / 1 g Polymerpulver). Anschließend wird der Teebeutel 3 Minuten lang bei 250 g zentrifugiert. Die Bestimmung der Flüssigkeitsmenge geschieht durch Auswägen des zentrifugierten Teebeutels.

Zur Ermittlung der CRC bei den Vergleichsversuchen wird anstelle der Polymerenmischung das Hydrogel-formende Polymer alleine eingesetzt (0.2000 ± 0.0050 g).

b) Absorption unter Druck (AUL Absorbency Under Load) (0.7 psi)

Die Meßzelle zur Bestimmung der AUL 0.7 psi stellt ein Plexiglas-Zylinder mit einem Innendurchmesser von 60 mm und einer Höhe von 50 mm dar, der an der Unterseite einen angeklebten Edelstahl-Siebboden mit einer Maschenweite von 36 $\mu$m besitzt. Zu der Meßzelle gehört weiterhin eine Plastikplatte mit einem Durchmesser von 59 mm und ein Gewicht, welches zusammen mit der Plastikplatte in die Meßzelle hineingestellt werden kann. Das Gewicht der Plastikplatte und das Gewicht betragen zusammen 1345 g. Zur Durchführung der Bestimmung der AUL 0.7 psi wird das Gewicht des leeren Plexiglas-Zylinders und der Plastikplatte ermittelt und als $W_0$ notiert. Dann werden 0.900 ± 0.005 g eines erfindungsgemäßen Polymergemisches, z. B. Polymerenmischung bestehend aus 0.81 g Hydrogel-formendem Polymer (Kornfraktion 106 - 850 $\mu$m) und 0.09 g Copolymere aus $C_2$ - $C_8$ Olefinen oder Styrolen mit Anhydriden (Kornfraktion 100 - 400 $\mu$m) in den Plexiglas-Zylinder eingewogen und möglichst gleichmäßig auf dem Edelstahl-Siebboden verteilt. Anschließend wird die Plastikplatte vorsichtig in den Plexiglas-Zylinder hineingelegt und die gesamte Einheit gewogen; das Gewicht wird als $W_a$ notiert. Nun wird das Gewicht auf die Plastikplatte in dem Plexiglas-Zylinder gestellt. In die Mitte der Petrischale mit einem Durchmesser von 200 mm und einer Höhe von 30 mm wird eine keramische Filterplatte mit einem Durchmesser von 120 mm und einer Porosität 0 gelegt und soviel 0.9 Gew.%ige Natriumchloridlösung eingefüllt, dass die Flüssigkeitsoberfläche mit der Filterplattenoberfläche abschließt, ohne dass die Oberfläche der Filterplatte benetzt wird. Anschliessend wird ein rundes Filterpapier mit einem Durchmesser von 90 mm und einer Porengröße < 20 $\mu$m (S&S 589 Schwarzband von Schleicher & Schüll) auf die keramische Platte gelegt. Der die Polymerenmischung enthaltende Plexiglas-Zylinder wird mit Plastikplatte und Gewicht nun auf das Filterpapier gestellt und dort für 60 Minuten belassen. Nach dieser Zeit wird die komplette Einheit aus der Petrischale vom Filterpapier herausgenommen und anschließend das Gewicht aus dem Plexiglas-Zylinder entfernt. Der gequollenes Hydrogelmischung enthaltende Plexiglas-Zylinder wird zusammen mit der Plastikplatte ausgewogen und das Gewicht als $W_b$ notiert.

Die Absorption unter Druck (AUL) wird wie folgt berechnet:

$$\texttt{AUL 0.7 psi [g/g]} = [W_b - W_a] \ / \ [W_a - W_0]$$

Die AUL 0.5 psi wird mit einem entsprechend verringerten Gewicht der Plastikplatte gemessen.

Zur Ermittlung der AUL 0.7 psi bzw. 0.5 psi bei den Vergleichsversuchen wird anstelle der Polymerenmischung das Hydrogel-formende Polymer alleine eingesetzt (0.9000 ± 0.005 g).

c) Saline Flow Conductivity (SFC)

Die Testmethode zur Bestimmung der SFC ist beschrieben in U.S. 5 599 335.

d) Messung des pH-Wertes der Hydrogel-formenden Polymere

100 ml 0,9 Gew.-%ige NaCl-Lösung werden in einem 150 ml-Becherglas mit Hilfe eines Magnetrührers mit moderater Geschwindigkeit gerührt, so dass durch die Rührung keine Luft in die Lösung eingezogen wird. Zu dieser Lösung werden 0,5 ± 0,001 g des zu messenden Polymers gegeben und 10 Minuten lang gerührt. Nach 10 Minuten wird der pH der Lösung mit Hilfe einer pH-Glaselektrode gemessen, wobei der Wert erst dann abgelesen wird, wenn er stabil ist, frühestens jedoch nach 1 Minute.

e) Messung der Pufferkapazität der Hydrogel-formenden Polymere

Zur Ermittlung der Pufferkapazität der Hydrogel-formenden Polymere wurden 0,5 ± 0,001 g Hydrogel-formendes Polymer bzw. Polymermischung in 100 ml 0,9 Gew.-%ige NaCl-Lösung in einem 150 ml-Becherglas vorgelegt und mit Hilfe eines Magnetrührers mit moderater Geschwindigkeit gerührt, so dass durch die Rührung keine Luft in die Lösung eingezogen wird. Nach 10 Minuten wird der pH-Wert der Lösung erstmals mit Hilfe einer pH-Glaselektrode gemessen, wobei der Wert erst dann abgelesen wird, wenn er stabil ist, frühestens jedoch nach 1 Minute. Im folgenden wurde dann 0,1 molare NaOH-Lösung so zugegeben, daß alle 5 Minuten 0,05 ml an 0,1 molarer NaOH-Lösung unter weiterem Rühren zudosiert wurden. Im Laufe der Zugabe wurde der pH-Wert der Mischung laufend überprüft. Zur Auswertung gelangte der pH-Wert vor Zugabe der 0,1 molaren NaOH-Lösung, sowie der pH-Wert nach 6 Stunden.

Beispiele

Die in den Beispielen erhaltenen Polymerenmischungen zeichnen sich im Gegensatz zu den in den Vergleichsbeispielen erhaltenen Polymeren durch eine Kombination von Absorptionsquantität und Quellgeschwindigkeit aus und weisen eine hohe Flüssigkeitspermeabilität, sowie verbesserte Geruchsbindungseigenschaften auf. Sie sind deshalb in hervorragender Weise als Absorptionsmittel für Wasser und wässrige Flüssigkeiten, insbesondere von Körperflüssigkeiten, wie z. B. Urin oder Blut, geeignet, beispielsweise in Hygieneartikeln wie z. B. Baby- und Erwachsenenwindeln, Damenbinden, Tampons und dergleichen.

Die nachfolgenden Beispiele sollen die Erfindung näher erläutern.

Vergleichsbeispiel 1

a) In einem 40 1-Plastikeimer werden 6,9 kg reine Acrylsäure mit 20 kg entionisiertem Wasser verdünnt. Zu dieser Lösung fügt man 33 g Pentaerythritoltriallylether unter Rühren hinzu, und inertisiert den verschlossenen Eimer durch Durchleiten von Stickstoff. Die Polymerisation wird dann durch Zugabe von 0,4 g Wasserstoffperoxid, gelöst in 40 ml entionisiertem Wasser, und 0,2 g Ascorbinsäure, gelöst in 40 ml entionisiertem Wasser, gestartet. Nach Beendigung der Reaktion wird das Gel mechanisch zerkleinert, und mit soviel Natronlauge versetzt bis ein Neutralisierungsgrad von 75 mol% bezogen auf die eingesetzte Acrylsäure erreicht wird. Das neutralisierte Gel wird dann auf einem Walzentrockner getrocknet, mit einer Stiftmühle gemahlen, und schließlich bei 150 - 850 $\mu$m abgesiebt.

b) Das unter a) hergestellte Grundpolymer wurde in einem Lödige-Labormischer mit, bezogen auf Grundpolymer, 2,9 Gew.-% Vernetzer-Lösung, zusammengesetzt aus 49,56 Gewichtsanteilen Propandiol-1,2 49,56 Gewichtsanteilen entionisiertem Wasser und 0,88 Gewichtsanteilen Monoethylenglycoldiglycidylester (EDGE) besprüht. Anschließend wurde das feuchte Produkt in einen zweiten vorgeheizten Lödige-Labormischer überführt und bei 140°C für 60 Minuten getempert. Das getrocknete und auf Raumtemperatur abgekühlte Produkt wurde bei 850 $\mu$m abgesiebt.

Vergleichsbeispiel 2

a) In einem 40 1-Plastikeimer werden 6,9 kg reine Acrylsäure mit 20 kg entionisiertem Wasser verdünnt. Zu dieser Lösung fügt man 33 g Pentaerythritoltriallylether unter Rühren hinzu, und inertisiert den verschlossenen Eimer durch Durchleiten von Stickstoff. Die Polymerisation wird dann durch Zugabe von 0,4 g Wasserstoffperoxid, gelöst in 40 ml entionisiertem Wasser, und 0,2 g Ascorbinsäure, gelöst in 40 ml entionisiertem Wasser, gestartet. Nach Beendigung der Reaktion wird das Gel mechanisch zerkleinert, und mit soviel Natronlauge versetzt bis ein Neutralisierungsgrad von 75 mol% bezogen auf die eingesetzte Acrylsäure erreicht wird. Das neutralisierte Gel wird dann auf einem Walzentrockner getrocknet, mit einer Stiftmühle gemahlen, und schließlich bei 150 - 850 $\mu$m abgesiebt.
b) Das unter a) hergestellte Grundpolymer wurde in einem Lödige-Labormischer mit, bezogen auf Grundpolymer, 3,75 Gew.-% Vernetzer-Lösung, zusammengesetzt aus 33,3 Gewichtsanteilen Propandiol-1,2 63,5 Gewichtsanteilen entionisiertem Wasser und 3,2 Gewichtsanteilen EDGE sowie mit 0,12 Gewichtsanteilen einer 27 %igen, wässrigen Aluminiumsulfatlösung besprüht. Vernetzerlösung und Aluminiumsulfatlösung werden getrennt voneinander, aber zeitgleich über 2 Düsen aufgesprüht. Anschließend wurde das feuchte Produkt in einen zweiten vorgeheizten Lödige-Labormischer überführt und bei 140 °C für 60 Minuten getempert. Das getrocknete und auf Raumtemperatur abgekühlte Produkt wurde bei 850 $\mu$m abgesiebt.

Vergleichsbeispiel 3

In einem durch geschäumtes Kunststoffmaterial gut isolierten Polyethylengefäß mit einem Fassungsvermögen von 10 1 werden 3928 g VE-Wasser vorgelegt, 625 g Natriumhydrogencarbonat darin supendiert und 2000 g Acrylsäure unter Rühren so zufliessen lassen, dass kein Überschäumen durch einsetzende $CO_2$-Entwicklung eintritt.. Nacheinander werden eine Emulsion aus 1,3 g Sorbitanmonococoat in 100 g VE-Wasser und 8,1 g Allylmethacrylat zugesetzt und die Lösung durch Einleiten von Stickstoff weiter inertisiert. Dann erfolgt die Zugabe des Initiatorsystems, bestehend aus 1,66 g 2,2'-Azobisamidinopropan-dihydrochlorid, gelöst in 20g VE-Wasser, 3,33 g Kaliumperoxodisulfat, gelöst in 150 g VE-Wasser sowie 0,3 g Ascorbinsäure, gelöst in 25 g VE-Wasser nacheinander unter Rühren. Die Reaktionslösung wird danach ohne Rühren stehen gelassen, wobei durch die einsetzende Polymerisation, in deren Verlauf die Temperatur auf ca. 90 °C ansteigt, ein festes Gel entsteht. Dieses wird mittels eines Fleischwolfes mechanisch zerkleinert und im Umlufttrockenschrank auf einem Siebgewebe aus VA-Draht bei Temperaturen von 160 °C getrocknet, anschließend gemahlen und gesiebt.

Vergleichsbeispiel 4

TYLOSE VS 3790, ein Superabsorber der CASSELLA AG - Frankfurt am Main, gekennzeichnet durch einen pH von 5-5,5, hergesetellt analog Beispiel 7 des EP 0 316 792 B1 wurde im 20 g Maßstab in einem WARING-Blender ( modifizierter Aufsatz für Küchenmixer ) mit einer Oberflächennachvernetzungslösung versetzt ( aus 2 ml-Spritze aufgespritzt), bestehend aus 2,3 % Wasser /1,2 % Propandiol-1,2 / 0,2 % Ethylenglykoldiglycidylether (jeweils bezogen auf Polymer) und 1 Stunde bei 140 °C in einem Umlufttrockenschrank getempert.

Vergleichsbeispiel 5

In einen mittels Vakkumpumpe auf 980 mbar absolut evakuierten Laborkneter mit einem Arbeitsvolumen von 2 1 ( WERNER + PFLEIDERER ) wird eine zuvor separat hergestellte, auf. 25 °C abgekühlte und durch Einleiten von Stickstoff inertisierte Monomerlösung eingesaugt. Die Monomerlösung setzt sich wie folgt zusammen: 825,5 g VE-Wasser, 431 g Acrylsäure, 120,68 g NaOH 50%ig, 0,86 g Polyethylenglykol-400-diacrylat ( SARTOMER® 344 der Firma CRAY VALLEY ) Zur besseren Inertisierung wird der Kneter evakuiert und anschließend mit Stickstoff belüftet. Dieser Vorgang wird 3x wiederholt. Anschließend wird eine Lösung aus 1,2 g Natriumpersulfat, gelöst in 6,8 g VE-Wasser und nach weiteren 30 Sekunden eine weitere Lösung, bestehend aus 0,024 g Ascorbinsäure, gelöst in 4,8 g VE-Wasser eingesaugt. Es wird mit Stickstoff gespült. Ein auf 75 °C vorgeheizter Mantelheizkreislauf ( Bypass ) wird auf den Knetermantel umgestellt, die Rührerdrehzahl auf 96 UpM erhöht. Nach einsetzender Polymerisation und Erreichen von $T_{max}$ wird der Mantelheizkreislauf wieder auf Bypass umgestellt und 15 Minuten ohne Heizung/Kühlung nachpolymerisiert, anschließend gekühlt, das Produkt ausgetragen. und die entstandenen Gelpartikel auf Blechen mit Drahtnetzboden bei 160°C im Umlufttrockenschrank getrocknet, anschließend gemahlen und gesiebt.

1200 g des so erhaltenen Produktes der Kornverteilung 105-850 $\mu$m wurden in einem Pulvermischaggregat ( Loedige-Mischer ) mit einer homogenen Lösung, bestehend aus 17,58 g Wasser, 9,96 g Propandiol-1,2, 1,2 g Ethylenglykoldiglycidylether und 3,36 g einer 26,8 %igen, wässrigen Aluminiumsulfat-Lösung besprueht, in einen vorgewärmten und 2. Lödige-Mischer umgefüllt. Unter konstant gehaltenen Bedingungen bei 150 °C Manteltemperatur und einer Umdrehungszahl von 60 UpM erfolgte die Temperung über eine Zeitraumes von 70 Minuten. Der Mischer wurde entleert, das Produkt auf Raumtemperatur runtergekühlt und bei 850 $\mu$m abgesiebt.

Beispiel 1

5 Teile Pulver des Copolymers i-Butylen / Maleinsäureanhydrid (1/1) der Firma Kuraray Isoprene Chemical Company, Ltd,. (Tokio, Japan, Handelsmarke: ISOBAM®) und 95 Teile aus Vergleichsbeispiel 4 werden in einem Labor-Taumelmischer für 60 Minuten homogen vermischt.

Beispiel 2

10 Teile Pulver des Copolymers i-Butylen / Maleinsäureanhydrid (1/1) Firma Kuraray Isoprene Chemical Company, Ltd,. (Tokio, Japan, Handelsmarke: ISOBAM®) und 90 Teile aus Vergleichsbeispiel 4 werden in einem Labor-Taumelmischer für 60 Minuten homogen vermischt.

Beispiel 3

5 Teile Pulver des Copolymers i-Butylen / Maleinsäureanhydrid (1/1) der Firma Kuraray Isoprene Chemical Company, Ltd,. (Tokio, Japan, Handelsmarke: ISOBAM®) und 95 Teile aus Vergleichsbeispiel 2 werden in einem Labor-Taumelmischer für 60 Minuten homogen vermischt.

Beispiel 4

10 Teile Pulver des Copolymers i-Butylen / Maleinsäureanhydrid (1/1) der Firma Kuraray Isoprene Chemical Company, Ltd,. (Tokio, Japan, Handelsmarke: ISOBAM®) und 90 Teile aus Vergleichsbeispiel 2 werden in einem Labor-Taumelmischer für 60 Minuten homogen vermischt.

Beispiel 5

50 Teile Pulver der i-Butylen / Maleinsäureanhydrid Copolymer-Fasern, hergestellt gemäß Methode US 5,026,784 Example 1 Spalte 8 Zeile 24 und 50 Teile aus Vergleichsbeispiel 1 werden in einem Labor-Taumelmischer für 60 Minuten homogen vermischt.

Die Performancedaten der Beispiele zur Absorptionsleistung sind der Tabelle 1 zu entnehmen, die Geruchsbindungseigenschaften sind in Tabelle 2 mit dem Messwert pH-Wert nach 6 Stunden (Pufferkapazität) angenähert.

Tabelle 1

| Beispiel | pH | SFC x $10^{-7}$ cm$^3$s/g | CRC g/g | AUL 0.5 psi g/g | AUL 0.7 psi g/g |
|---|---|---|---|---|---|
| Vergleichs-beispiel 1 | 5,95 | 5 | 33,4 | 29,4 | 22,8 |
| Vergleichs-beispiel 2 | 6,1 | 15 | 29 | 26 | 23 |
| Vergleichs-beispiel 3 | 4,5 | 3 | 23 | 11 | 7 |
| Vergleichs-beispiel 4 | 5,4 | ≤ 1 | 42,0 | | 6,0 |
| Vergleichs-beispiel 5 | 4,47 | 13,8 | 20,7 | | 18,1 |
| Beispiel 1 | 5,4 | 9 | 42,0 | | 6,1 |
| Beispiel 2 | 5,4 | 14 | 40,8 | | 5,9 |
| Beispiel 3 | 6,1 | 18 | 28,7 | 25,5 | 23 |
| Beispiel 4 | 6,1 | 24 | 28,9 | 25,9 | 22,3 |
| Beispiel 5 | 6.4 | ≤ 1 | 41,8 | 28,7 | 20,2 |

Tabelle 2

| Beispiel | pH | pH-Wert nach 6 Stunden (Pufferkapazität) |
|---|---|---|
| Vergleichsbeispiel 1 | 5,95 | 6,6 |
| Vergleichsbeispiel 2 | 6,1 | 7,1 |
| Vergleichsbeispiel 3 | 4,5 | 4,7 |
| Vergleichsbeispiel 5 | 4,47 | 4,6 |
| Beispiel 1 | 5,4 | 5,5 |
| Beispiel 2 | 5,4 | 5,4 |
| Beispiel 3 | 6,1 | 6,2 |
| Beispiel 4 | 6,1 | 5,9 |

Die Ergebnisse zeigen, dass die erfindungsgemäßen Beispiele annähernd die gleiche Performance bzgl. der Absorptionsleistung aufweisen bei deutlich verbesserter Geruchsbindung. Letzteres konnte im Test durch die enorme Pufferkapazität bei der Titration mit 0,1 molarer NaOH-Lösung gezeigt werden. Vergleichsbeispiel 3 und Vergleichsbeispiel 5 weisen bei geringerem pH-Wert als Beispiel 1 und 2 zwar ebenfalls gute Geruchsbindung auf; die Performance vor allem hinschtlich der CRC ist jedoch deutlich schlechter.

Bei den Vergleichsbeispielen 1 und 2 handelt es sich um Hydrogel-formendes Material aus dem Stand der Technik, das bei pH-Werten von 5,95 bzw. 6,1 vor allem hinsichtlich der Absorptionsleistung unter Druckbelastung optimiert ist während die Geruchsbindungseigenschaften (Pufferkapazität) mäßig sind. Wird dieses Material allerdings mit Copolymer-Fasern vermischt (hier im Verhältnis 1:1 mit Vergleichsbeispiel 1), so sind Steigerungen bei der Retention zu erkennen.

**Patentansprüche**

1. Polymergemische enthaltend die Komponenten (i) wässrige Flüssigkeiten absorbierende Hydrogel-formende Polymere, hergestellt durch Polymerisation von olefinisch ungesättigten Carbonsäuren oder deren Derivaten, und (ii) Copolymere aus $C_2$ - $C_8$ Olefinen oder Styrolen mit Anhydriden, wobei das molare Verhältnis zwischen $C_2$-$C_8$ Olefinen bzw. Styrol und Anhydrid zwischen 3 zu 1 und 1 zu 3 liegt.

2. Polymergemische nach Anspruch 1, wobei die Komponente (i) granulär oder faserförmig vorliegt und die Komponente (ii) unabhängig davon granulär oder faserförmig vorliegt und optional die Komponente (ii) zusätzlich faserförmig oder granulär vorliegt.

3. Polymergemische nach Anspruch 1, wobei die Komponente (ii) auf die Komponente (i) als Polymer aufgesprüht wird oder als Momomermischung aufgesprüht wird mit anschließender Polymerisation.

4. Polymergemische nach einem der Ansprüche 1 bis 3, wobei die Komponente (i) auf Polyacrylatbasis vorliegt.

5. Polymergemische nach einem der Ansprüche 1 bis 4, wobei die Komponente (ii) granulär vorliegt.

6. Polymergemische nach einem der Ansprüche 1 bis 5, wobei die Komponente (ii) unverseift vorliegt.

7. Polymergemische nach einem der Ansprüche 1 bis 6, wobei die Anhydrid-Komponente der Komponente (ii) Maleinsäureanhydrid ist und die olefinische oder Styrol Komponente ausgewählt wird aus einer oder mehreren der folgenden Verbindungen: Isobutylen, Vinylacetat, Ethylen oder Styrol.

8. Polymergemische nach einem der Ansprüche 1 bis 7, wobei die Komponente (i) als gepfropftes Produkt, insbesondere auf Carboxymethylcellulose, vorliegt.

9. Polymergemische nach einem der Ansprüche 1 bis 8, wobei die Komponente (i) zu 99,7 Gew.% bis 85 Gew.% und die Komponente (ii) zu 0,3 Gew.% bis 15 Gew % vorliegt.

10. Hygieneartikel enthaltend Polymerenmischungen nach einem der Ansprüche 1 bis 9.

11. Verwendung von Polymerenmischungen nach einem der Ansprüche 1 bis 9 als Absorptionsmittel für wässrige Flüssigkeiten bei verminderter Geruchsbildung.

**Claims**

1. Polymer mixtures comprising the components (i) hydrogel-forming polymers capable of absorbing aqueous fluids and prepared by polymerization of olefinically unsaturated carboxylic acids or derivatives thereof, and (ii) copolymers of $C_2$-$C_8$ olefins or styrenes with anhydrides in a molar ratio between $C_2$-$C_8$ olefins or styrene and anhydride in the range from 3:1 to 1:3.

2. Polymer mixtures according to claim 1, wherein the component (i) is granular or fibrous and the component (ii) is independently granular or fibrous and optionally the component (ii) is additionally fibrous or granular.

3. Polymer mixtures according to claim 1, wherein the component (ii) is sprayed onto the component (i) as a polymer or as a monomer mixture with subsequent polymerization.

4. Polymer mixtures according to any of claims 1 to 3, wherein the component (i) is based on polyacrylate.

5. Polymer mixtures according to any of claims 1 to 4, wherein the component (ii) is granular.

**6.** Polymer mixtures according to any of claims 1 to 5, wherein the component (ii) is unhydrolyzed.

**7.** Polymer mixtures according to any of claims 1 to 6, wherein the anhydride component of the component (ii) is maleic anhydride and the olefinic or styrene component is selected from one or more of the following compounds: isobutylene, vinyl acetate, ethylene and styrene.

**8.** Polymer mixtures according to any of claims 1 to 7, wherein the component (i) is a grafted product, especially on carboxymethylcellulose.

**9.** Polymer mixtures according to any of claims 1 to 8, wherein the component (i) is present in a fraction in the range from 99.7% by weight to 85% by weight and the component (ii) is present in a fraction in the range from 0.3% by weight to 15% by weight.

**10.** Hygiene articles comprising polymer mixtures according to any of claims 1 to 9.

**11.** The use of polymer mixtures according to any of claims 1 to 9 as an absorbent for aqueous fluids that provides reduced odor formation.

**Revendications**

**1.** Mélanges polymères contenant les composants : (i) des polymères formateurs d'hydrogel absorbant des liquides aqueux, préparés par polymérisation d'acides carboxyliques oléfiniquement insaturés ou de leurs dérivés, et (ii) des copolymères d'oléfines en $C_2$-$C_8$ ou de styrènes avec des anhydrides, le rapport molaire entre les oléfines en $C_2$-$C_8$ ou respectivement le styrène et l'anhydride étant compris entre 3 à 1 et 1 à 3.

**2.** Mélanges polymères suivant la revendication 1, dans lesquels le composant (i) se présente sous forme granuleuse ou fibreuse et le composant (ii) se présente indépendamment de cela sous forme granuleuse ou fibreuse et éventuellement le composant (ii) se présente en supplément sous forme fibreuse ou granuleuse.

**3.** Mélanges polymères suivant la revendication 1, dans lesquels le composant (ii) est pulvérisé sur le composant (i) comme polymère ou est pulvérisé comme mélange de monomères avec polymérisation ultérieure.

**4.** Mélanges polymères suivant l'une des revendications 1 à 3, dans lesquels le composant (i) se présente à base de polyacrylate.

**5.** Mélanges polymères suivant l'une des revendications 1 à 4, dans lesquels le composant (ii) se présente sous forme granuleuse.

**6.** Mélanges polymères suivant l'une des revendications 1 à 5, dans lesquels le composant (ii) se présente sous forme non saponifiée.

**7.** Mélanges polymères suivant l'une des revendications 1 à 6, dans lesquels le composant anhydride du composant (ii) est de l'anhydride maléique et le composant oléfinique ou de styrène est choisi parmi un ou plusieurs des composés suivants : isobutylène, acétate de vinyle, éthylène ou styrène.

**8.** Mélanges polymères suivant l'une des revendications 1 à 7, dans lesquels le composant (i) se présente sous la forme d'un produit greffé, en particulier sur de la carboxyméthylcellulose.

**9.** Mélanges polymères suivant l'une des revendications 1 à 8, dans lesquels le composant (i) est présent à 99,7 % en poids jusqu'à 85 % en poids et le composant (ii) à 0,3 % en poids jusqu'à 15 % en poids.

**10.** Articles d'hygiène contenant des mélanges polymères suivant l'une des revendications 1 à 9.

**11.** Utilisation de mélanges polymères suivant l'une des revendications 1 à 9, en tant qu'agents absorbants pour des liquides aqueux dans la réduction de la formation d'odeur.